# DEMANDE DE BREVET EUROPEEN

(11) **EP 1 010 691 A2**
(43) Date de publication de la demande: **21.06.2000**
(21) Numéro de dépôt: 99403191.2
(22) Date de dépôt: 17.12.1999
(51) Int. Cl.: C07C 271/22, C07D 213/82, C07D 209/18, C07C 243/34, C07D 209/42, C07C 311/19, C07C 311/29, C07C 311/46, C07C 311/49, A61K 31/15, A61K 31/18, A61K 31/33

(54) **Dérives de l'hydrazide, leur procédé de préparation et les compositions pharmaceutiques qui les contiennent**

(30) Priorité: 17.12.1998 ES 9802626
(71) Demandeur: ADIR ET COMPAGNIE, 92415 Courbevoie Cédex (FR)
(72) Inventeur: Monge Vega, Antonio, 31180 Cizur Menor, Navarra (ES); Aldana Moraza, Ignacio, 31008 Pamplona, Navarra (ES); Caignard, Daniel-Henri, 78230 Le Pecq (FR); Duhault, Jacques, 78290 Croissy sur Seine (FR); Boutin, Jean, 92150 Suresnes (FR); Della Zuana, Odile, 93220 Romainville (FR)
(74) Mandataire: Ruiz, Nicolas

(57) **Abrégé**

Composés de formule (I) :

R-NH-A-CO-NH-NH-(W)ₙ - Z (I)

dans laquelle :
◆ n vaut 0 ou 1,
◆ W représente un groupement -CO- ou un groupement S(O)ᵣ dans lequel r vaut 0, 1 ou 2,
◆ Z représente un groupement choisi parmi aryle, arylalkyle, hétéroaryle et hétéroarylalkyle éventuellement substitués,
◆ R représente un groupement choisi parmi :

   Z₁-T-CO-, Z₁-O-T-CO-, Z₁-T-O-CO-, Z₁-T-S(O)_{q}-

   dans lesquels Z₁, T et q sont tels que définis dans la description,
◆ A représente un groupement alkylène, alkénylène ou alkynylène de 3 à 8 atomes de carbone, alkylènecycloalkylène, cycloalkylènealkylène, alkylènecycloalkylènealkylène, alkylènearylène, arylènealkylène, alkylènearylènealkylène, un groupement 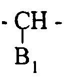 dans lequel B1 est tel que défini dans la description, ou bien A forme avec l'atome d'azote adjacent un groupement 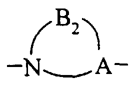 tel que défini dans la description.

Médicaments.

## Description

La présente invention concerne de nouveaux dérivés de l'hydrazide, leur procédé de préparation et les compositions pharmaceutiques qui les contiennent.

Des dérivés de l'hydrazide ont été décrits dans la littérature (J. Org. Chem., 1971, 36, 1580) sans qu'aucune propriété pharmacologique ne soit mentionnée. D'autres composés de structure voisine entrent dans la composition de films photographiques (JP 02008833), ou bien ont été utilisés pour la formation de polymères servant à préparer des membranes semiperméables (J. Appl. Polym. Sci., 1992, 44, 1383).

Les composés de la présente invention possèdent une structure originale qui leur confère une grande affinité pour les récepteurs du Neuropeptide Y.

Des ligands de ces récepteurs ont été décrits récemment. A titre d'exemple, on peut citer des composés peptidiques cycliques (WO 9400486), des aminoacides dérivés de l'arginine (WO 9417035), ou bien des composés non peptidiques possédant un groupement guanidine (EP 448765, J. Med. Chem., 1994, 37, 2242).

Le Neuropeptide Y (NPY) est un peptide de 36 acides aminés, proche du peptide YY (PYY) et des polypeptides pancréatiques (PP). A l'origine isolé du cerveau de porc (Proc. Natl. Acad. Sci., 1982, 79, 5485), le NPY est largement distribué chez les mammifères au niveau du système nerveux central et périphérique. Ce neurotransmetteur est présent en fortes concentrations dans les fibres nerveuses du cerveau, mais aussi du coeur, des ganglions sympathiques, des vaisseaux sanguins et des muscles lisses du vas deferens et du tractus gastrointestinal. Il est responsable d'effets physiologiques divers qui s'exercent par l'intermédiaire de récepteurs spécifiques (Y). Ces derniers forment un groupe hétérogène dont 6 sous-types ont été identifiés jusqu'à aujourd'hui : Y₁ à Y₆ (Pharmacological Reviews, 1998, 50, 143). Le NPY est impliqué dans le comportement alimentaire, en stimulant fortement la prise de nourriture (Proc. Natl. Acad. Sci., 1985, 82, 3940), ou en exerçant un rôle de régulateur sur l'axe HPA (Hypothalamique-Pituritaire-Surrenal) (J. of Neuroendocrinol., 1995, 7, 273). Il présente également des propriétés anxiolytiques et sédatives (Neuropsychopharmacology, 1993, 8, 357), un fort pouvoir vasoconstricteur (Eur. J. Pharmacol., 1984, 85, 519) qui induit une augmentation de la pression sanguine, et possède aussi un effet sur le rythme circadien (Neuroscience and biobehavioral reviews. 1995, 19, 349).

Les composés de l'invention, outre le fait qu'ils soient nouveaux, possèdent une structure qui leur confère une grande affinité pour les récepteurs du NPY. Ils pourront donc être utilisés dans le traitement des pathologies nécessitant un ligand des récepteurs du NPY, en particulier dans le traitement des pathologies liées à des troubles du comportement alimentaire ou de la balance énergétique, telles que le diabète, l'obésité, la boulimie, l'anorexie mentale, mais également dans le traitement de l'hypertension artérielle, de l'anxiété, des dépressions, de l'épilepsie, des dysfonctionnements sexuels et des troubles du sommeil.

La présente invention concerne les composés de formule (I) :

R - NH - A - CO - NH - NH - (W)ₙ - Z (I)

dans laquelle :
◆ n vaut 0 ou 1,
◆ W représente un groupement -CO- ou un groupement S(O)ᵣ dans lequel r vaut 0, 1 ou 2,
◆ Z représente un groupement choisi parmi aryle éventuellement substitué, arylalkyle éventuellement substitué, hétéroaryle éventuellement substitué et hétéroarylalkyle éventuellement substitué,
◆ R représente un groupement choisi parmi :

   Z₁-T-CO-

   Z₁-O-T-CO-

   Z₁-T-O-CO-

   Z₁-T-S(O)_{q}-

   dans lesquels :
   - Z₁ représente un groupement aryle éventuellement substitué, arylalkyle éventuellement substitué, hétéroaryle éventuellement substitué ou hétéroarylalkyle éventuellement substitué,
   - T représente une liaison a ou un groupement alkylène, alkénylène ou alkynylène,
   - q représente un entier égal à 0, 1 ou 2,
◆ A représente un groupement alkylène linéaire ou ramifié de 3 à 8 atomes de carbone, alkénylène linéaire ou ramifié de 3 à 8 atomes de carbone, alkynylène linéaire ou ramifié de 3 à 8 atomes de carbone, alkylènecycloalkylène, cycloalkylènealkylène, alkylènecycloalkylènealkylène, alkylènearylène, arylènealkylène, alkylènearylènealkylène, un groupement 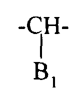 dans lequel B₁ représente un groupement aryle éventuellement substitué, arylalkyle éventuellement substitué, hétéroaryle éventuellement substitué, hétéroarylalkyle éventuellement substitué, ou bien A forme avec l'atome d'azote un groupement 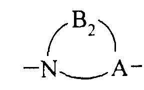 dans lequel B₂ représente un système mono ou bicyclique de 5 à 11 chaînons saturé ou insaturé comportant éventuellement de 1 à 3 hétéroatomes supplémentaires choisis parmi azote, oxygène et soufre,

à la condition que lorsque simultanément n vaut 0, A représente un groupement 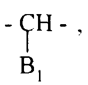 B₁ étant un groupement benzyle, et Z représente un groupement phényle éventuellement substitué, alors R est différent d'un groupement benzoyle,
leurs énantiomères, diastéréoisomères, ainsi que leurs sels d'addition à un acide ou à une base pharmaceutiquement acceptable,
étant entendu que,
le terme alkyle désigne un groupement linéaire ou ramifié de 1 à 6 atomes de carbone,
le terme alkylène désigne un radical bivalent, linéaire ou ramifié, contenant de 1 à 6 atomes de carbones, sauf précisions contraires,
le terme alkénylène désigne un radical bivalent linéaire ou ramifié, contenant de 2 à 6 atomes de carbones et 1 à 3 doubles liaisons, sauf précisions contraires,
le terme alkynylène désigne un radical bivalent, linéaire ou ramifié, contenant de 2 à 6 atomes de carbone et 1 à 3 triples liaisons, sauf précisions contraires,
le terme aryle désigne un groupement phényle, napthyle, dihydronapthyle, ou tétrahydronapthyle, le terme arylène désignant un groupement bivalent du même type,
le terme hétéroaryle désigne un groupement mono ou bicyclique insaturé ou partiellement insaturé, de 5 à 11 chaînons, contenant de 1 à 4 hétéroatomes choisis parmi azote, oxygène et soufre,
le terme alkylènecycloalkylène représente un groupement -A₁-A₂-, le terme cycloalkylène-alkylène représente un groupement -A₂-A₁-, et le terme alkylènecycloalkylènealkylène représente un groupement -A₁-A₂-A₁, le terme alkylènearylène représente un groupement -A₁-A₃-, le terme arylènealkylène représente un groupement -A₃-A₁-, le terme alkylènearylènealkylène représente un groupement -A₃-A₁-A₃, dans lesquels Ai est un groupement alkylène tel que défini précédemment, A₂ est un groupement cycloalkylène (C₄-C₈), et A₃ est un groupement arylène tel que défini précédemment,
l'expression éventuellement substitué affectée aux termes aryle, arylalkyle, hétéroaryle, hétéroarylalkyle, signifie que ces groupements sont substitués sur leur partie cyclique par 1 à 5 substituants, identiques ou différents, choisis parmi alkyle (C₁-C₆) linéaire ou ramifié, alkoxy (C₁-C₆) linéaire ou ramifié, halogène, hydroxy, perhalogénoalkyle (C₁-C₆) linéaire ou ramifié, nitro, acyle (C₁-C₆) linéaire ou ramifié, alkylsulfonyle (C₁-C₆) linéaire ou ramifié, et amino (éventuellement substitué par un ou deux groupements alkyle (C₁-C₆) linéaire ou ramifié, ou acyle (C₁-C₆) linéaire ou ramifié).

Parmi les acides pharmaceutiquement acceptables, on peut citer à titre non limitatif les acides chlorhydrique, bromhydrique, sulfurique, phosphonique, acétique, trifluoroacétique, lactique, pyruvique, malonique, succinique, glutarique, fumarique, tartrique, maléïque, citrique, ascorbique, oxalique, méthane sulfonique, camphorique, etc...

Parmi les bases pharmaceutiquement acceptables, on peut citer à titre non limitatif l'hydroxyde de sodium, l'hydroxyde de potassium, la triéthylamine, la tertbutylamine, etc...

De façon avantageuse, l'invention concerne les composés de formule (I) pour lesquels R représente un groupement Z₁-T-CO, Z₁ étant préférentiellement un groupement aryle éventuellement substitué, et T étant préférentiellement un groupement alkylène ou une liaison.

Un autre aspect avantageux de l'invention concerne les composés de formule (I) pour lesquels R représente un groupement Z₁-O-T-CO, Z₁ étant préférentiellement un groupement aryle éventuellement substitué, et T étant préférentiellement un groupement alkylène ou une liaison.

Un autre aspect avantageux de l'invention concerne les composés de formule (I) pour lesquels R représente un groupement Z₁-T-O-CO, Z₁ étant préférentiellement un groupement aryle éventuellement substitué, et T étant préférentiellement un groupement alkylène ou une liaison.

Un autre aspect avantageux de l'invention concerne les composés de formule (I) pour lesquels R représente un groupement Z₁-T-S(O)_{q}-, Z₁ étant préférentiellement un groupement aryle éventuellement substitué, et T étant préférentiellement un groupement alkylène ou une liaison, et q étant préférentiellement 2.

Des composés préférés de l'invention sont ceux pour lesquels W représente un groupement -CO-.

D'autres composés préférés de l'invention sont ceux pour lesquels W représente un groupement SO₂.

Dans les composés préférés de l'invention, Z représente un groupement choisi parmi aryle éventuellement substitué et hétéroaryle éventuellement substitué.

Des composés préférés de l'invention sont ceux pour lesquels A représente un groupement 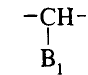 dans lequel B₁ est un groupement arylalkyle éventuellement substitué (par exemple un groupement benzyle ou tolylméthyle).

D'autres composés préférés de l'invention sont ceux pour lesquels A représente un groupement alkylènecycloalkylène (par exemple méthylènecyclohexylène).

D'autres composés préférés de l'invention sont ceux pour lesquels A représente un groupement alkylènearylène (par exemple méthylènephénylène).

Dans les composés de l'invention, les groupements cycliques 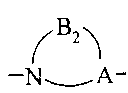 sont avantageusement choisis parmi les groupements pyrrolidine, perhydroindole et pipéridine.

Les groupements aryles préférés de l'invention sont les groupements phényle ou naphtyle.

De façon particulièrement avantageuse, l'invention concerne les composés de formule (I) pour lesquels W représente un groupement -CO-, Z représente un groupement choisi parmi aryle éventuellement substitué, et hétéroaryle éventuellement substitué, R représente un groupement choisi parmi Z₁-T-CO-, Z₁-O-T-CO-, Z₁-T-O-CO-, Z₁-T-S(O)_{q}- dans lesquels Z₁ est préférentiellement un groupement aryle éventuellement substitué, ou hétéroaryle éventuellement substitué, T représente un groupement alkylène (par exemple méthylène), et q vaut 2, et A représente un groupement alkylènecycloalkylène, un groupement 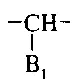 dans lequel B₁ est un groupement arylalkyle éventuellement substitué, ou bien A forme avec l'atome d'azote adjacent un groupement pyrrolidine, perhydroindole ou pipéridine.

Parmi les composés préférés de l'invention, on peut citer les :
- N2-({4-[(2-benzoylhydrazino)carbonyl]cyclohexyl}méthyl)-2-naphtalènesulfonamide
- N1-({4-[(2-benzoylhydrazino)carbonyl]cyclohexyl}méthyl)-1-(2-nitrobenzène) sulfonamide
- N1-[1-Benzyl-2-oxo-2-(2-phénylhydrazino)éthyl]-1-(4-chlorobenzène)sulfonamide

La présente invention concerne également le procédé de préparation des composés de formule (I) caractérisé en ce que l'on utilise comme produit de départ un composé de formule (II) :

H₂N - A - COOH (II)

dans laquelle A est tel que défini dans la formule (I),
qui est condensé en milieu basique sur un dérivé halogéné de formule (III) :

R - Cl (III)

dans laquelle R est tel que défini dans la formule (I),
pour conduire à un composé de formule (IV) :

R - NH - A - COOH (IV)

dans laquelle R et A sont tels que définis précédemment,
composé (IV) qui est condensé, en présence d'un agent de couplage, sur une hydrazine monosubstituée de formule (V),

H₂N-NH-(W)ₙ-Z (V)

dans laquelle n, W et Z sont tels que définis dans la formule (I),
pour conduire aux composés de formule (I) :

R - NH - A - CO - NH - NH - (W)ₙ - Z (I)

dans laquelle R, A, n, W et Z sont tels que définis précédemment,
composé de formule (I) :
- qui peut être, le cas échéant, purifié selon une technique classique de purification
- dont on sépare, le cas échéant, les isomères selon une technique classique de séparation,
- que l'on transforme, si on le souhaite, en ses sels d'addition à un acide ou à une base pharmaceutiquement acceptable.

La présente invention a également pour objet les compositions pharmaceutiques renfermant comme principe actif au moins un composé de formule (I), seul ou en combinaison avec un ou plusieurs excipients ou véhicules inertes non toxiques.

Parmi les compositions pharmaceutiques selon l'invention on pourra citer plus particulièrement celles qui conviennent pour l'administration orale, parentérale, nasale, les comprimés simples ou dragéifiés, les comprimés sublinguaux, les gélules, les tablettes, les suppositoires, les crèmes, pommades, gels dermiques, etc...

La posologie utile varie selon l'âge et le poids du patient, la nature et la sévérité de l'affection ainsi que la voie d'administration. Celle-ci peut être orale, nasale, rectale ou parentérale. D'une manière générale, la posologie unitaire s'échelonne entre 0,05 et 500 mg pour un traitement en 1 à 3 prises par 24 heures.

Les exemples suivants illustrent l'invention et ne la limitent en aucune façon. Les structures des composés décrits ont été confirmées par les techniques spectroscopiques usuelles. Les produits de départ utilisés sont des produits connus ou préparés selon des modes opératoires connus.

### EXEMPLE 1 : N-[1-Benzyl-2-oxo-2-(2-phénylhydrazino)éthyl]carbamate de benzyle

### Stade a : Acide 2-benzyloxycarbonylamino-3-phénylpropanoique

A une solution de 80 mmol (13,2 g) de Phénylalanine dans 20 ml de soude aqueuse 4M, refroidie à 0°C, sont ajoutées 82 mmol (13,9 g) de chloroformiate de benzyle et 20 ml de soude aqueuse 4M, sur une période de 30 mn. La solution revient à température ambiante pendant 1 heure. Le milieu réactionnel est extrait à l'éther. La phase aqueuse est acidifiée à pH = 2 par une solution diluée d'acide chlorhydrique. Le précipité formé est filtré et lavé pour conduire au composé attendu.

### Stade b : N-[1-Benzyl-2-oxo-2-(2-phénylhydrazino)éthyl]carbamate de benzyle

A une solution de 2,85 mmol (0,8 g) du composé décrit au stade précédent et de 3,25 mmol (0,43 g) de HOBT dans 20 ml de dichlorométhane, refroidie à 0°C sont ajoutées 3,25 mmol (0,63 g) d'EDC. La réaction est agitée à 0°C pendant 1 heure puis 3,25 mmol (0,32 ml) de phénylhydrazine sont ajoutées. La réaction est agitée à 0°C pendant 1 heure, puis 24 heures à température ambiante. Le milieu réactionnel est filtré et le filtrat est concentré. Le résidu obtenu est repris dans l'éther (15 ml) et 10 ml d'eau sont ajoutés. Le précipité obtenu est filtré, lavé à l'eau puis à l'éther pour conduire au composé attendu.
*Point de fusion : 175-177°C*

| *Microanalyse elementaire :* | | | |
|---|---|---|---|
| | *C* | *H* | *N* |
| *% calculé* | *70,95* | *5,91* | *10,79* |
| *% trouvé* | *71.27* | *5,90* | *10,71* |

### EXEMPLE 2 : N-[2(2-Benzoylhydrazino)-1-benzyl-2-oxoéthyl]carbamate de benzyle

Le produit attendu est obtenu selon le procédé décrit dans l'exemple 1 en remplaçant, au stade b, la phénylhydrazine par la phénylhydrazide.
*Point de fusion : 172-174°C*

| *Microanalyse élémentaire :* | | | |
|---|---|---|---|
| | *C* | *H* | *N* |
| *% calculé* | *69,06* | *5,52* | *10,07* |
| *% trouvé* | *69,14* | *5,71* | *9,92* |

### EXEMPLE 3 : N-[1-Benzyl-2-oxo-2-(2-nicotinoylhydrazino)éthyl]carbamate de benzyle

Le produit attendu est obtenu selon le procédé décrit dans l'exemple 1 en remplaçant, au stade b, la phénylhydrazine par la 3-pyridinecarbohydrazide.

| *Microanalyse élémentaire :* | | | |
|---|---|---|---|
| | *C* | *H* | *N* |
| *% calculé* | *66,03* | *5,26* | *13,40* |
| *% trouvé* | *66,00* | *5,72* | *13,68* |

### EXEMPLE 4 : N-{1-Benzyl-2-[2-(3-indolyl)acétyl]-2-oxoéthyl}carbamate de benzyle

Le produit attendu est obtenu selon le procédé décrit dans l'exemple 1 en remplaçant, au stade b, la phénylhydrazine par la 3-indolylacétohydrazide.

| *Microanalyse élémentaire :* | | | |
|---|---|---|---|
| | *C* | *H* | *N* |
| *% calculé* | *68,94* | *5,53* | *11,91* |
| *% trouvé* | *69,23* | *5,81* | *11,57* |

### EXEMPLE 5 : N1-[1-Benzyl-2-oxo-2-(2-phénylhydrazino)éthyl]-2-phénoxyacétamide

### Stade a : Acide 2-[(2-phénoxyacétyl)amino]-3-phénylpropanoïque

Une solution de 6,6 mmol (1,0 g) d'acide phénoxyacétique dans 15 ml de dioxane est traitée par 49,3 mmol (3,6 ml) de chlorure de thionyle. Le milieu réactionnel est agité à température ambiante pendant 2 heures, puis concentré. Le résidu, repris dans 10 ml de dichlorométhane ainsi que 7,9 mmol (0,3 g) de soude dans 10 ml d'eau, sont ajoutés successivement à une solution de 7,6 mmol (1,25 g) de Phénylalanine et 7,6 mmol (0,3 g) de soude dans 10 ml d'eau, en maintenant la température à 10°C. Le milieu réactionnel est ensuite agité 1 heure à température ambiante. Après décantation, la phase aqueuse est lavée par du dichlorométhane, puis acidifiée à pH = 2 par une solution diluée d'acide chlorhydrique. Le précipité formé est filtré et recristallisé dans l'eau pour conduire au produit attendu.

### Stade b : N1-[1-Benzyl-2-oxo-2-(2-phénylhydrazino)éthyl]-2-phénoxyacétamide

A une solution de 3,35 mmol (1 g) du composé décrit au stade précédent et de 3,7 mmol (0,56 g) de HOBT dans 15 ml de dichlorométhane, refroidie à 0°C, sont ajoutées 3,7 mmol (0,71 g) d'EDC. Après une heure à 0°C, une solution de 3,7 mmol (0,4 g) de phénylhydrazine dans 10 ml de dichlorométhane est ajoutée. La réaction est agitée à 0°C pendant 1 heure et ensuite pendant 24 heures à température ambiante. La phase organique est lavée à l'eau, séchée sur sulfate de sodium et concentrée. Le résidu obtenu est lavé dans l'éther, puis filtré pour conduire au composé attendu,
*Point de fusion : 172-175°C*

| *Microanalyse élémentaire* : | | | |
|---|---|---|---|
| | *C* | *H* | *N* |
| *% calculé* | *70,95* | *5,91* | *10,80* |
| *% trouvé* | *70,91* | *6,06* | *10,68* |

### EXEMPLE 6 : N1-[2-(2-Benzoylhydrazino)-1-benzyl-2-oxoéthyl]-2-phénoxyacétamide

Le produit attendu est obtenu selon le procédé décrit dans l'exemple 5 en remplaçant, au stade b, la phénylhydrazine par la phénylhydrazide.

| *Microanalyse élémentaire* : | | | |
|---|---|---|---|
| | *C* | *H* | *N* |
| *% calculé* | *69,06* | *5,12* | *10,07* |
| *% trouvé* | *68,88* | *5,52* | *9,84* |

### EXEMPLE 7 : N1-{1-Benzyl-2-[2-(2-indolylcarbonyl)hydrazino]-2-oxoéthyl}-2-phénoxyacétamide

Le produit attendu est obtenu selon le procédé décrit dans l'exemple 5 en remplaçant, au stade b, la phénylhydrazine par la 2-indolecarbohydrazide.

| *Microanalyse élémentaire :* | | | |
|---|---|---|---|
| | *C* | *H* | *N* |
| *% calculé* | *68,42* | *5,26* | *12,28* |
| *% trouvé* | *67,97* | *5,44* | *12,42* |

### EXEMPLE 8 : N1-[1-Benzyl-2-oxo-2-(2-nicotinoylhydrazino)éthyl]-2-phénoxyacétamide

Le produit attendu est obtenu selon le procédé décrit dans l'exemple 5 en remplaçant, au stade b, la phénylhydrazine par la 3-pyridinecarbohydrazide.

| *Microanalyse élémentaire :* | | | |
|---|---|---|---|
| | *C* | *H* | *N* |
| *% calculé* | *64,29* | *5,36* | *12,50* |
| *% trouvé* | *64,06* | *5,34* | *12,34* |

### EXEMPLE 9 : N2-[1-Benzyl-2-oxo-2-(2-phénylhydrazino)éthyl]-2-indolecarboxamide

### Stade a : Acide 2-(2-indolylcarbonylamino)-3-phénylpropanoïque

Le produit attendu est obtenu selon le procédé décrit dans l'exemple 5, stade a, en remplaçant l'acide phénoxyacétique par l'acide 2-indolecarboxylique.

### Stade b : N2-[1-Benzyl-2-oxo-2-(2-phénylhydrazino)éthyl]-2-indolecarboxamide

Le produit attendu est obtenu selon le procédé décrit dans l'exemple 5, stade b, en utilisant comme produit de départ le composé décrit au stade précédent.

| *Microanalyse élémentaire :* | | | |
|---|---|---|---|
| | *C* | *H* | *N* |
| *% calculé* | *72,36* | *5,53* | *14,07* |
| *% trouvé* | *72,05* | *5,82* | *14,60* |

### EXEMPLE 10 : N2-[1-Benzyl-2-oxo-2-(2-phénylhydrazino)éthyl]-2-naphtalènesulfonamide

### Stade a : Acide 2-[(2-napthylsulfonyl)amino]-3-phénylpropanoïque

Une solution de 7,6 mmol (1,71 g) de chlorure de 2-naphtylsulfonyle dans 10 ml de dichlorométhane et une solution de 7,9 mmol (0,32 g) de soude dans 10 ml d'eau sont ajoutées successivement et lentement à 7,6 mmol (1,25 g) de Phénylalanine et 7,6 mmol (0,3 g) de soude dans 10 ml d'eau. La réaction est agitée 1 heure à température ambiante. Après décantation, la phase aqueuse est lavée par du dichlorométhane et acidifiée à pH = 2 par une solution diluée d'acide chlorhydrique. Après extraction au dichlorométhane, la phase organique est séchée sur sulfate de sodium et concentrée pour conduire au produit attendu.

### Stade b : N2-[1-Benzyl-2-oxo-2-(2-phénylhydrazino)éthyl]-2-naphtalènesulfonamide

Le produit attendu est obtenu selon le procédé décrit dans l'exemple 1, stade b, en utilisant comme produit de départ le composé décrit au stade précédent.

| *Microanalyse élémentaire :* | | | |
|---|---|---|---|
| | *C* | *H* | *N* |
| *% calculé* | *67,41* | *5,17* | *9,44* |
| *% trouvé* | *67,03* | *5,34* | *9,57* |

### EXEMPLE 11 : N2-[2-(2-Benzoylhydrazino)-1-benzyl-2-oxoéthyl]-2-naphtalènesulfonamide

Le produit attendu est obtenu selon le procédé décrit dans l'exemple 1, stade b, en utilisant comme produit de départ le composé décrit dans l'exemple 10, stade a, et en remplaçant la phénylhydrazine par la phénylhydrazide.
*Point de fusion : 239-240°C*

| *Microanalyse élémentaire :* | | | |
|---|---|---|---|
| | *C* | *H* | *N* |
| *% calculé* | *65,96* | *4,86* | *8,87* |
| *% trouvé* | *65,55* | *4,99* | *8,88* |

### EXEMPLE 12 : N2-[1-Benzyl-2-oxo-2-(2-phénylhydrazino)éthyl]-2-naphtylamide

Le produit attendu est obtenu selon le procédé décrit dans l'exemple 1 en remplaçant au stade a le chloroformiate de benzyle par le chlorure de naphtoyle.

| *Microanalyse élémentaire :* | | | |
|---|---|---|---|
| | *C* | *H* | *N* |
| *% calculé* | *76,28* | *5,62* | *10,27* |
| *% trouvé* | *76,25* | *5,76* | *10,03* |

### EXEMPLE 13 : N2-[2-(2-Benzoylhydrazino)-1-benzyl-2-oxoéthyl]-2-naphtylamide

Le produit attendu est obtenu selon le procédé décrit dans l'exemple 1 en remplaçant au stade a le chloroformiate de benzyle par le chlorure de napthoyle et au stade b, la phénylhydrazine par la phénylhydrazide.

| *Microanalyse élémentaire :* | | | |
|---|---|---|---|
| | *C* | *H* | *N* |
| *% calculé* | *74,17* | *5,26* | *9,61* |
| *% trouvé* | *74,21* | *5,38* | *9,49* |

### EXEMPLE 14 : N2-[1-Benzyl-2-oxo-2-(2-nicotinoylhydrazino)éthyl]-2-naphtylamide

Le produit attendu est obtenu selon le procédé décrit dans l'exemple 1 en remplaçant au stade a le chloroformiate de benzyle par le chlorure de napthoyle et au stade b, la phénylhydrazine par la 3-pyridinecarbohydrazide.

| *Microanalyse élémentaire :* | | | |
|---|---|---|---|
| | *C* | *H* | *N* |
| *% calculé* | *71,23* | *5,02* | *12,79* |
| *% trouvé* | *70,97* | *5,38* | *12,61* |

### EXEMPLE 15 : N2-{1-Benzyl-2-[2-(2-indolcarbonyl)hydrazino]-2-oxoéthyl}-2-naphtylamide

Le produit attendu est obtenu selon le procédé décrit dans l'exemple 1 en remplaçant au stade a le chloroformiate de benzyle par le chlorure de napthoyle et au stade b, la phénylhydrazine par la 2-indolecarbohydrazide.
*Point de fusion : 214-215°C*

| *Microanalyse élémentaire :* | | | |
|---|---|---|---|
| | *C* | *H* | *N* |
| *% calculé* | *73,11* | *5,25* | *11,76* |
| *% trouvé* | *72,98* | *5,16* | *11,75* |

### EXEMPLE 16 : N1-[1-Benzyl-2-oxo-2-(2-phénylhydrazino)éthyl]-1-benzènesulfonamide

### Stade a : Acide 2-(phénylsulfonylamino)-3-phénylpropanoïque

Un mélange de 151 mmol (26,6 g) de chlorure de benzènesulfonyle et de 50 ml de soude aqueuse 4M est ajouté à une solution de 37,8 mmol (6,25 g) de Phénylalanine dans 50 ml de soude aqueuse 4M. Le mélange réactionnel est agité à température ambiante pendant 24 heures. La solution est ensuite acidifiée à pH = 2 par de l'acide chlorhydrique dilué et extraite par de l'éther. La phase organique est séchée sur sulfate de magnésium et concentrée. Le résidu obtenu est recristallisé dans l'éthanol pour conduire au composé attendu.

### Stade b : N1-[1-Benzyl-2-oxo-2-(2-phénylhydrazino)éthyl]-1-benzènesulfonamide

Le produit attendu est obtenu selon le procédé décrit dans l'exemple 1, stade b, en utilisant comme produit de départ le composé décrit au stade précédent.
*Point de fusion : 162-163°C*

| *Microanalyse élémentaire* : | | | |
|---|---|---|---|
| | *C* | *H* | *N* |
| *% calculé* | *63,80* | *5,32* | *10,63* |
| *% trouvé* | *63,03* | *5,38* | *10.38* |

### EXEMPLE 17 : N1-[2-(2-Benzoylhydrazino)-1-benzyl-2-oxoéthyl]1-benzènesulfonamide

Le produit attendu est obtenu selon le procédé décrit dans l'exemple 1, stade b, en utilisant comme produit de départ le composé de l'exemple 16, stade a, et en remplaçant la phénylhydrazine par la phénylhydrazide.
*Point de fusion : 200°C*

| *Microanalyse élémentaire :* | | | |
|---|---|---|---|
| | *C* | *H* | *N* |
| *% calculé* | *62,41* | *4,96* | *9,93* |
| *% trouvé* | *62,59* | *5,06* | *9,84* |

### EXEMPLE 18 : N1-[1-Benzyl-2-oxo-2-(2-phénylhydrazino)éthyl]-1-(4-chlorobenzène) sulfonamide

Le produit attendu est obtenu selon le procédé décrit dans l'exemple 10, en remplaçant, au stade a, le chlorure de 2-naphtylsulfonyle par le chlorure de 4-chlorobenzènesulfonyle.
*Point de fusion : 174-175°C*

| *Microanalyse élémentaire :* | | | |
|---|---|---|---|
| | *C* | *H* | *N* |
| *% calculé* | *58,67* | *4,66* | *9,78* |
| *% trouvé* | *58,63* | *4,77* | *9,70* |

### EXEMPLE 19 : N1-[2-(2-Benzoylhydrazino)-1-benzyl-2-oxoéthyl]-1-(4-chlorobenzène) sulfonamide

Le produit attendu est obtenu selon le procédé décrit dans l'exemple 10, en remplaçant, au stade a, le chlorure de 2-naphtylsulfonyle par le chlorure de 4-chlorobenzènesulfonyle, et au stade b, la phénylhydrazine par la phénylhydrazide.
*Point de fusion : 192-193°C*

| *Microanalyse élémentaire :* | | | |
|---|---|---|---|
| | *C* | *H* | *N* |
| *% calculé* | *57,70* | *4,37* | *9,18* |
| *% trouvé* | *57,50* | *4,43* | *9,14* |

### EXEMPLE 20 : N1-[1-Benzyl-2-oxo-2-(2-phénylhydrazino)éthyl]-1-(3,4-dichloro-benzène)sulfonamide

Le produit attendu est obtenu selon le procédé décrit dans l'exemple 16, en remplaçant, au stade a, le chlorure de benzènesulfonyle par le chlorure de 3,4-dichlorobenzènesulfonyle.
*Point de fusion : 164-165°C*

| *Microanalyse élémentaire :* | | | |
|---|---|---|---|
| | *C* | *H* | *N* |
| *% calculé* | *54,32* | *4,12* | *9,05* |
| *% trouvé* | *54,14* | *4,16* | *8,87* |

### EXEMPLE 21 : N1-[2-(2-Benzoylhydrazino)-1-benzyl-2-oxoéthyl]-1-(3,4-dichlorobenzène)sulfonamide

Le produit attendu est obtenu selon le procédé décrit dans l'exemple 16, en remplaçant, au stade a, le chlorure de benzènesulfonyle par le chlorure de 3,4-dichlorobenzènesulfonyle, et au stade b, la phénylhydrazine par la phénylhydrazide.
*Point de fusion : 200-201°C*

| *Microanalyse élémentaire :* | | | |
|---|---|---|---|
| | *C* | *H* | *N* |
| *% calculé* | *53,66* | *3,86* | *8,54* |
| *% trouvé* | *53,45* | *3,93* | *8,28* |

### EXEMPLE 22 : N-[1-(4-Méthoxybenzyl)-2-oxo-2-(2-phénylhydrazino)éthyl]carbamate de benzyle

Le produit attendu est obtenu selon le procédé décrit dans l'exemple 1, en remplaçant au stade a la Phénylalanine par la O-Méthyltyrosine.
*Point de fusion : 182-185°C*

| *Microanalyse élémentaire :* | | | |
|---|---|---|---|
| | *C* | *H* | *N* |
| *% calculé* | *68,74* | *5,97* | *10,02* |
| *% trouvé* | *68,56* | *6,11* | *9,81* |

### EXEMPLE 23 : N-[2-Benzoylhydrazino-1-(4-méthoxybenzyl)-2-oxoéthyl]carbamate de benzyle

Le produit attendu est obtenu selon le procédé décrit dans l'exemple 1, en remplaçant au stade a la Phénylalanine par la O-Méthyltyrosine, et au stade b la phénylhydrazine par la phénylhydrazide.
*Point de fusion: 208-209°C*

| *Microanalyse élémentaire :* | | | |
|---|---|---|---|
| | *C* | *H* | *N* |
| *% calculé* | *67,11* | *5,59* | *9,40* |
| *% trouvé* | *67,19* | *5,74* | *9,32* |

### EXEMPLE 24 : N-{2-[2-(2-indolylcarbonyl)hydrazino]-1-(4-méthoxybenzyl)-2-oxoéthyl}carbamate de benzyle

Le produit attendu est obtenu selon le procédé décrit dans l'exemple 1, en remplaçant au stade a la Phénylalanine par la O-Méthyltyrosine, et au stade b la phénylhydrazine par la 2-indolecarbohydrazide.
*Point de fusion : 185-186°C*

| *Microanalyse élémentaire :* | | | |
|---|---|---|---|
| | *C* | *H* | *N* |
| *% calculé* | *66,67* | *5,35* | *11,52* |
| *% trouvé* | *66,40* | *5,43* | *12,87* |

### EXEMPLE 25 : N-[1-(4-Méthoxybenzyl)-2-(2-nicotinoylhydrazino)-2-oxoéthyl] carbamate de benzyle

Le produit attendu est obtenu selon le procédé décrit dans l'exemple 1, en remplaçant au stade a la Phénylalanine par la O-Méthyltyrosine, et au stade b la phénylhydrazine par la 3-pyridinecarbohydrazide.

| *Microanalyse élémentaire :* | | | |
|---|---|---|---|
| | *C* | *H* | *N* |
| *% calculé* | *64,29* | *5,36* | *12,50* |
| *% trouvé* | *64,43* | *5,52* | *12,14* |

### EXEMPLE 26 : N-(2-{2-[2-(3-indolyl)acétyl]hydrazino}-1-(4-méthoxybenzyl)-2- oxoéthyl)carbamate de benzyle

Le produit attendu est obtenu selon le procédé décrit dans l'exemple 1, en remplaçant au stade a la Phénylalanine par la O-Méthyltyrosine, et au stade b la phénylhydrazine par la 3-indolylacétohydrazide.
*Point de fusion : 194-195°C*

| *Microanalyse élémentaire :* | | | |
|---|---|---|---|
| | *C* | *H* | *N* |
| *% calculé* | *67,20* | *5,60* | *11,19* |
| *% trouvé* | *67,01* | *5,59* | *11,12* |

### EXEMPLE 27 : N1-[1-(4-Méthoxybenzyl)-2-oxo-2-(2-phénylhydrazino)éthyl]-2- phénoxyacétamide

Le produit attendu est obtenu selon le procédé décrit dans l'exemple 5, en remplaçant, au stade a, la Phénylalanine par la O-Méthyltyrosine.

| *Microanalyse élémentaire :* | | | |
|---|---|---|---|
| | *C* | *H* | *N* |
| *% calculé* | *68,74* | *5,97* | *10,02* |
| *% trouvé* | *68,37* | *6,06* | *9,86* |

### EXEMPLE 28 : N1-[2-(2-Benzoylhydrazino)-1-(4-méthoxybenzyl)-2-oxoéthyl]-2-phénoxyacétamide

Le produit attendu est obtenu selon le procédé décrit dans l'exemple 5, en remplaçant, au stade a, la Phénylalanine par la O-Méthyltyrosine, et au stade b la phénylhydrazine par la phénylhydrazide.

| *Microanalyse élémentaire* : | | | |
|---|---|---|---|
| | *C* | *H* | *N* |
| *% calculé* | *67,11* | *5,59* | *9,40* |
| *% trouvé* | *67,19* | *5,81* | *9,60* |

### EXEMPLE 29 : N1-[1-(4-Méthoxybenzyl)-2-(2-nicotinoylhydrazino)-2-oxoéthyl]-2- phénoxyacétamide

Le produit attendu est obtenu selon le procédé décrit dans l'exemple 5, en remplaçant, au stade a, la Phénylalanine par la O-Méthyltyrosine, et au stade b la phénylhydrazine par la 3-pyridinecarbohydrazide.

| *Microanalyse élémentaire :* | | | |
|---|---|---|---|
| | *C* | *H* | *N* |
| *% calculé* | *64,29* | *5,36* | *12,50* |
| *% trouvé* | *64,06* | *5,34* | *12,34* |

### EXEMPLE 30 : N2-[1-(4-Méthoxybenzyl)-2-oxo-2-(2-phénylhydrazino)éthyl]-2- naphtalènesulfonamide

Le produit attendu est obtenu selon le procédé décrit dans l'exemple 10, en remplaçant la Phénylalanine par la O-Méthyltyrosine.
*Point de fusion : 210-212°C*

| *Microanalyse élémentaire :* | | | |
|---|---|---|---|
| | *C* | *H* | *N* |
| *% calculé* | *64,47* | *5,37* | *8,87* |
| *% trouvé* | *64,17* | *5,25* | *8,56* |

### EXEMPLE 31 : N2-[2-(2-Benzoylhydrazino)-1-(4-méthoxybenzyl)-2-oxoéthyl]-2- naphtalènesulfonamide

Le produit attendu est obtenu selon le procédé décrit dans l'exemple 10, en remplaçant, au stade a, la Phénylalanine par la O-Méthyltyrosine, et au stade b, la phénylhydrazine par la phénylhydrazide.
*Point de fusion : 246-247°C*

| *Microanalyse élémentaire :* | | | |
|---|---|---|---|
| | *C* | *H* | *N* |
| *% calculé* | *63,28* | *5,07* | *8,20* |
| *% trouvé* | *63,14* | *5,01* | *8,55* |

### EXEMPLE 32 : N2-[1-(4-Méthoxybenzyl)-2-oxo-2-(2-phénylhydrazino)éthyl]-2- naphtylamide

Le produit attendu est obtenu selon le procédé décrit dans l'exemple 1, en remplaçant, au stade a, la Phénylalanine par la O-Méthyltyrosine et le chloroformiate de benzyle par le chlorure de naphtoyl.

| *Microanalyse élémentaire :* | | | |
|---|---|---|---|
| | C | H | N |
| *% calculé* | *73,80* | *5,70* | *9,57* |
| *% trouvé* | *73,53* | *5,62* | *9,54* |

### EXEMPLE 33 : N2-[1-(4-Méthoxybenzyl)-2-(2-nicotinoylhydrazino)-2-oxoéthyl]-2- naphtylamide

Le produit attendu est obtenu selon le procédé décrit dans l'exemple 1, en remplaçant, au stade a, la Phénylalanine par la O-Méthyltyrosine et le chloroformiate de benzyle par le chlorure de naphtoyl, et au stade b, en remplaçant la phénylhydrazide par la 3-pyridinecarbohydrazide.

| *Microanalyse élémentaire :* | | | |
|---|---|---|---|
| | *C* | *H* | *N* |
| *% calculé* | *69,23* | *5,13* | *11,97* |
| *% trouvé* | *69,25* | *5,13* | *11,72* |

### EXEMPLE 34 : N1-[1-(4-Méthoxybenzyl)-2-oxo-2-(2-phénylhydrazino)éthyl]-1- benzènesulfonamide

Le produit attendu est obtenu selon le procédé décrit dans l'exemple 16, en remplaçant, au stade a, la L-Phénylalanine par la O-Méthyltyrosine.
*Point de fusion : 163-164°C*

| *Microanalyse élémentaire :* | | | |
|---|---|---|---|
| | *C* | *H* | *N* |
| *% calculé* | *62,12* | *5,41* | *9,88* |
| *% trouvé* | *61,71* | *5,54* | *9,89* |

### EXEMPLE 35 : N1-[2-(2-Benzoylhydrazino)-1-(4-méthoxybenzyl)-2-oxoéthyl]-1- benzènesulfonamide

Le produit attendu est obtenu selon le procédé décrit dans l'exemple 16, en remplaçant, au stade a, la L-Phénylalanine par la O-Méthyltyrosine, et au stade b, la phénylhydrazine par la phénylhydrazide.

| *Microanalyse élémentaire :* | | | |
|---|---|---|---|
| | *C* | *H* | *N* |
| *% calculé* | *60,85* | *5,07* | *6,26* |
| *% trouvé* | *60,81* | *5,23* | *9,20* |

### EXEMPLE 36 : N1-[1-(4-Méthoxybenzyl)-2-oxo-2-(2-phénylhydrazino)éthyl]-1-(4- chlorobenzène)sulfonamide

Le produit attendu est obtenu selon le procédé décrit dans l'exemple 10, en remplaçant au stade a la Phénylalanine par la O-Méthyltyrosine et le chlorure de 2-napthylsulfonyle par le chlorure de 4-chlorobenzènesulfonyle.
*Point de fusion : 181-183°C*

| *Microanalyse élémentaire :* | | | |
|---|---|---|---|
| | *C* | *H* | *N* |
| *% calculé* | *56,31* | *4,92* | *9,39* |
| *% trouvé* | *56,64* | *4,85* | *9,02* |

### EXEMPLE 37 : N1-[2-(2-Benzoylhydrazino)-1-(4-méthoxybenzyl)-2-oxoéthyl]-1-(4- chlorobenzène)sulfonamide

Le produit attendu est obtenu selon le procédé décrit dans l'exemple 10, en remplaçant au stade a la Phénylalanine par la O-Méthyltyrosine et le chlorure de 2-napthylsulfonyle par le chlorure de 4-chlorobenzènesulfonyle, et au stade b, en remplaçant la phénylhydrazine par la phénylhydrazide.

| *Microanalyse élémentaire :* | | | |
|---|---|---|---|
| | *C* | *H* | *N* |
| *% calculé* | *56,61* | *4,54* | *8,61* |
| *% trouvé* | *56,82* | *4,55* | *8,61* |

### EXEMPLE 38 : N1-[1-(4-Méthoxybenzyl)-2-oxo-2-(2-phénylhydrazino)éthyl]-1-(3,4- dichlorobenzène)sulfonamide

Le produit attendu est obtenu selon le procédé décrit dans l'exemple 16, en remplaçant, au stade a, la Phénylalanine par la O-Méthyltyrosine et le chlorure de benzène sulfonyle par le chlorure de 3,4-dichlorobenzène sulfonyle.
*Point de fusion : 153-154°C*

| *Microanalyse élémentaire :* | | | |
|---|---|---|---|
| | *C* | *H* | *N* |
| *% calculé* | *53,44* | *4,25* | *8,50* |
| *% trouvé* | *53,80* | *4,38* | *8,30* |

### EXEMPLE 39 : N1-[2-(2-Benzoylhydrazino)-1-(4-méthoxybenzyl)-2-oxoéthyl]-1-(3,4- dichlorobenzène)sulfonamide

Le produit attendu est obtenu selon le procédé décrit dans l'exemple 16, en remplaçant, au stade a, la Phénylalanine par la O-Méthyltyrosine et le chlorure de benzène sulfonyle par le chlorure de 3,4-dichlorobenzène sulfonyle, et au stade b, la phénylhydrazine par la phénylhydrazide.
*Point de fusion : 214-215°C*

| *Microanalyse élémentaire :* | | | |
|---|---|---|---|
| | *C* | *H* | *N* |
| *% calculé* | *52,87* | *4,03* | *8,05* |
| *% trouvé* | *52,60* | *4,09* | *8,17* |

### EXEMPLE 40 : N2-[6-Oxo-6-(2-phénylhydrazino)hexyl]-2-naphtalènesulfonamide

### Stade a : Acide 6-(2-naphtylsulfonyl)amino]hexanoïque

A une solution de 15,2 mmol (2 g) d'acide 6-aminocaproïque dans 15 ml de soude aqueuse 4M, sont ajoutés successivement 30,5 mmol (6,42 g) de chlorure de 2-naphtylsulfonyle et 15 ml de soude aqueuse 4M. Le milieu réactionnel est agité à température ambiante pendant 24 heures. La solution est ensuite acidifiée à pH = 2 par l'acide chlorhydrique concentré, et extraite au dichlorométhane. La phase organique est séchée sur sulfate de sodium, concentrée et le résidu obtenu est recristallisé dans l'hexane pour conduire au composé attendu.

### Stade b : N2-[6-Oxo-6-(2-phénylhydrazino)hexyl]-2-naphtalènesulfonamide

Le produit attendu est obtenu selon le procédé décrit dans l'exemple 1, stade b, en utilisant comme produit de départ le composé décrit au stade précédent.

| *Microanalyse élémentaire :* | | | |
|---|---|---|---|
| | *C* | *H* | *N* |
| *% calculé* | *64,16* | *6,08* | *10,21* |
| *% trouvé* | *64,23* | *5,88* | *9,95* |

### EXEMPLE 41 : N2-[6-(2-Benzoylhydrazino)-6-oxohexyl]-2-naphtalènesulfonamide

Le produit attendu est obtenu selon le procédé décrit dans l'exemple 40 en remplaçant, au stade b, la phénylhydrazine par la phénylhydrazide.

| *Microanalyse élémentaire :* | | | |
|---|---|---|---|
| | *C* | *H* | *N* |
| *% calculé* | *62,79* | *5,69* | *9,56* |
| *% trouvé* | *62,50* | *5,70* | *9,49* |

### EXEMPLE 42 : N2-[6-(2-Benzoylhydrazino)-6-oxohexyl]-2-(2-nitrophényl) sulfonamide

Le produit attendu est obtenu selon le procédé décrit dans l'exemple 40 en remplaçant, au stade a, le chlorure de 2-napthylsulfonyle par le chlorure de 2-nitrophénylsulfonyle.
*Point de fusion* : *106°C*

| *Microanalyse élémentaire :* | | | |
|---|---|---|---|
| | *C* | *H* | *N* |
| *% calculé* | *52,53* | *5,10* | *12,89* |
| *% trouvé* | *52,61* | *5,14* | *12,76* |

### EXEMPLE 43 : N2-({4-[(2-Phénylhydrazino)carbonyl]cyclohexyl}méthyl)-2- naphtalènesulfonamide

Le produit attendu est obtenu selon le procédé décrit dans l'exemple 40 en remplaçant, au stade a, l'acide 6-aminocaproïque par l'acide 4-(aminométhyl)cyclohexanecarboxylique.

| *Microanalyse elementaire : (x½ H*_{*2*}*O)* | | | |
|---|---|---|---|
| | *C* | *H* | *N* |
| *% calculé* | *64,50* | *6,27* | *9,41* |
| *% trouvé* | *64,54* | *6,04* | *9,22* |

### EXEMPLE 44 : N2-({4-[(2-Benzoylhydrazino)carbonyl]cyclohexyl}méthyl)-2-naphtalènesulfonamide

Le produit attendu est obtenu selon le procédé décrit dans l'exemple 40 en remplaçant, au stade a, l'acide 6-aminocaproïque par l'acide 4-(aminométhyl)cyclohexanecarboxylique, et au stade b la phénylhydrazine par la phénylhydrazide.

| *Microanalyse élémentaire : (x½ H*_{*2*}*O)* | | | |
|---|---|---|---|
| | *C* | *H* | *N* |
| *% calculé* | *63.22* | *5,90* | *8,85* |
| *% trouvé* | *63,59* | *5,68* | *8,76* |

### EXEMPLE 45 : N2-[(4-{[2-(2-Indolylcarbonyl)hydrazino]carbonyl}cyclohexyl) méthyl]-2-naphtalènesulfonamide

Le produit attendu est obtenu selon le procédé décrit dans l'exemple 40 en remplaçant, au stade a, l'acide 6-aminocaproïque par l'acide 4-(aminométhyl)cyclohexanecarboxylique, et au stade b, la phénylhydrazine par la 2-indolylcarbohydrazide.

| *Microanalyse élementaire (x½ H*_{*2*}*O)* | | | |
|---|---|---|---|
| | *C* | *H* | *N* |
| *% calculé* | *63,11* | *5,45* | *10,90* |
| *% trouvé* | *63,11* | *5,72* | *11,10* |

### EXEMPLE 46 : N2-({4-[(2-Nicotinoylhydrazino)carbonyl]cyclohexyl}méthyl)-2- naphtalènesulfonamide

Le produit attendu est obtenu selon le procédé décrit dans l'exemple 40 en remplaçant, au stade a, l'acide 6-aminocaproïque par l'acide 4-(aminométhyl)cyclohexanecarboxylique, et au stade b, la phénylhydrazine par la 3-pyridinecarbohydrazide.
*Point de fusion : 204-205°C*

| *Microanalyse élémentaire :* | | | |
|---|---|---|---|
| | *C* | *H* | *N* |
| *% calculé* | *61,76* | *5,57* | *12,01* |
| *% trouvé* | *62,15* | *5,68* | *11,88* |

### EXEMPLE 47 : N2-{(4-({2-[2-(3-Indolyl)-acétyl]hydrazino}carbonyl)cyclohexyl] méthyl}-2-naphtalènesulfonamide

Le produit attendu est obtenu selon le procédé décrit dans l'exemple 40 en remplaçant, au stade a, l'acide 6-aminocaproïque par l'acide 4-(aminométhyl)cyclohexanecarboxylique, et au stade b, la phénylhydrazine par la 3-indolylacétohydrazide.

| *Microanalyse élémentaire :* | | | |
|---|---|---|---|
| | C | H | N |
| *% calculé* | *62,61* | *5,77* | *10,44* |
| *% trouvé* | *62,11* | *5,99* | *10,41* |

### EXEMPLE 48 : N1-({4-[(2-Phénylhydrazino)carbonyl]cyclohexyl}méthyl)-1-benzylsulfonamide

Le produit attendu est obtenu selon le procédé décrit dans l'exemple 40 en remplaçant, au stade a, l'acide 6-aminocaproïque par l'acide 4-(aminométhyl)cyclohexanecarboxylique, et le chlorure de 2-naphtylsulfonyle par le chlorure de benzène sulfonyle.
*Point de fusion : 194-196°C*

| *Microanalyse élémentaire :* | | | |
|---|---|---|---|
| | *C* | *H* | *N* |
| *% calculé* | *61,99* | *6,50* | *10,84* |
| *% trouvé* | *62,81* | *6,60* | *10,83* |

### EXEMPLE 49 : N1-({4-[(2-Benzoylhydrazino)carbonyl]cyclohexyl}méthyl)-1- benzènesulfonamide

Le produit attendu est obtenu selon le procédé décrit dans l'exemple 40 en remplaçant, au stade a, l'acide 6-aminocaproïque par l'acide 4-(aminométhyl)cyclohexanecarboxylique, et le chlorure de 2-naphtylsuflonyle parle chlorure de benzène sulfonyle, et au stade b, la phénylhydrazine par la phénylhydrazide.

| *Microanalyse éléméntaire :* | | | |
|---|---|---|---|
| | *C* | *H* | *N* |
| *% calculé* | *60,70* | *6,06* | *10,11* |
| *% trouvé* | *60,25* | *6,24* | *10,07* |

### EXEMPLE 50 : N1-({4-[(2-Nicotinoylhydrazino)carbonyl]cyclohexyl}méhtyl)-1-benzènesulfonamide

Le produit attendu est obtenu selon le procédé décrit dans l'exemple 40 en remplaçant, au stade a, l'acide 6-aminocaproïque par l'acide 4-(aminométhyl)cyclohexanecarboxylique, et le chlorure de 2-naphtylsuflonyle par le chlorure de benzène sulfonyle, et au stade b, la phénylhydrazine par la 3-pyridinecarbohydrazide.
*Point de fusion : 213-215°C*

| *Microanalyse élémentaire : (x½ H*_{*2*}*O)* | | | |
|---|---|---|---|
| | C | H | N |
| *% calculé* | *56,41* | *5,64* | *13,16* |
| *% trouvé* | *56,45* | *5,74* | *13,56* |

### EXEMPLE 51 : N1-[(4-{[2-(4-Chlorophényl)hydrazino]carbonyl}cyclohexyl)méthyl]-1-benzènesulfonamide

Le produit attendu est obtenu selon le procédé décrit dans l'exemple 40 en remplaçant, au stade a, l'acide 6-aminocaproïque par l'acide 4-(aminométhyl)cyclohexanecarboxylique, et le chlorure de 2-naphtylsuflonyle par le chlorure de benzène sulfonyle, et au stade b, la phénylhydrazine par la 4-chlorophénylhydrazine.

| *Microanalyse élémentaire :* | | | |
|---|---|---|---|
| | *C* | *H* | *N* |
| *% calculé* | *56,93* | *5,73* | *9,96* |
| *% trouvé* | *56,91* | *5,82* | *9,86* |

### EXEMPLE 52 : N1-[(4-{2-(4-Chlorobenzoyl)hydrazino]carbonyl}cyclohexyl)méthyl]-1-benzènesulfonamide

Le produit attendu est obtenu selon le procédé décrit dans l'exemple 40 en remplaçant, au stade a, l'acide 6-aminocaproïque par l'acide 4-(aminométhyl)cyclohexanecarboxylique, et le chlorure de 2-naphtylsuflonyle par le chlorure de benzène sulfonyle, et au stade b, la phénylhydrazine par la 4-chlorophénylhydrazide.

| *Microanalyse élémentaire :* | | | |
|---|---|---|---|
| | *C* | *H* | *N* |
| *% calculé* | *56,01* | *5,33* | *9,33* |
| *% trouvé* | *55,98* | *5,43* | *9,41* |

### EXEMPLE 53 : N1-({4-[(2-Phénylhydrazino)carbonyl]cyclohexyl}méthyl)-1-(2- nitrobenzène)sulfonamide

Le produit attendu est obtenu selon le procédé décrit dans l'exemple 40 en remplaçant, au stade a, l'acide 6-aminocaproïque par l'acide 4-(aminométhyl)cyclohexanecarboxylique et le chlorure de 2-naphtylsulfonyle par le chlorure de 2-nitrobenzènesulfonyle.

| *Microanalyse élémentaire :* | | | |
|---|---|---|---|
| | *C* | *H* | *N* |
| *% calculé* | *55,54* | *5,59* | *12,95* |
| *% trouvé* | *55,35* | *5,49* | *12,70* |

### EXEMPLE 54 : N1-({4-((2-Benzoylhydrazino)carbonyl]cyclohexyl}méthyl)-1-(2-nitrobenzène)sulfonamide

Le produit attendu est obtenu selon le procédé décrit dans l'exemple 40 en remplaçant, au stade a, l'acide 6-aminocaproïque par l'acide 4-(aminométhyl)cyclohexanecarboxylique et le chlorure de 2-naphtylsulfonyle par le chlorure de 2-nitrobenzènesulfonyle, et au stade b la phénylhydrazine par la phénylhydrazide.

| *Microanalyse élémentaire : (x½ H*_{*2*}*O)* | | | |
|---|---|---|---|
| | *C* | *H* | *N* |
| *% calculé* | *53,72* | *5,15* | *11,93* |
| *% trouvé* | *53,57* | *5,19* | *11,76* |

### EXEMPLE 55 : N1-{[4-(2-[2-(3-Indolyl)-acétyl]hydrazino}carbonyl)cyclohexyl] méthyl}-1-(2-nitrobenzène)sulfonamide

Le produit attendu est obtenu selon le procédé décrit dans l'exemple 40 en remplaçant, au stade a, l'acide 6-aminocaproïque par l'acide 4-(aminométhyl)cyclohexanecarboxylique et le chlorure de 2-naphtylsulfonyle par le chlorure de 2-nitrobenzènesulfonyle, et au stade b la phénylhydrazine par la 3-indolylacétohydrazide.

| *Microanalyse élémentaire : (x½ H*_{*2*}*O)* | | | |
|---|---|---|---|
| | *C* | *H* | *N* |
| *% calculé* | *55,11* | *5,17* | *13,39* |
| *% trouvé* | *55,13* | *5,14* | *13,35* |

### EXEMPLE 56 : N1-[(4-{2-(2-Indolylcarbonyl)hydrazino]carbonyl}cyclohexyl)méthyl]-1-(2-nitrobenzène)sulfonamide

Le produit attendu est obtenu selon le procédé décrit dans l'exemple 40 en remplaçant, au stade a, l'acide 6-aminocaproïque par l'acide 4-(aminométhyl)cyclohexanecarboxylique et le chlorure de 2-naphtylsulfonyle par le chlorure de 2-nitrobenzènesulfonyle, et au stade b la phénylhydrazine par la 2-indolylcarbohydrazide.
*Point de fusion* : *249-251°C*

| *Microanalyse élémentaire :* | | | |
|---|---|---|---|
| | *C* | *H* | *N* |
| *% calculé* | *55,27* | *5,01* | *14,02* |
| *% trouvé* | *55,02* | *5,28* | *14,39* |

### EXEMPLE 57 : N1-({4-[(2-Benzoylhydrazino)carbonyl]cyclohexyl}méthyl)-1-(2-nitro-4-trifluorométhylphényl)sulfonamide

Le produit attendu est obtenu selon le procédé décrit dans l'exemple 40 en remplaçant, au stade a, l'acide 6-aminocaproïque par l'acide 4-(aminométhyl)cyclohexanecarboxylique et, le chlorure de 2-naphtylsulfonyle par le chlorure de 2-nitro-4-trifluorométhylphénylsuflonyle, et au stade b, la phénylhydrazine par le phénylhydrazide.

| *Microanalyse élémentaire :* | | | |
|---|---|---|---|
| | *C* | *H* | *N* |
| *% calculé* | *50,00* | *4,36* | *10,61* |
| *% trouvé* | *49,97* | *4,46* | *10,68* |

### EXEMPLE 58 : N1-({4-[(2-Phénylhydrazino)carbonyl]cyclohexyl}méthyl)-1-(4-bromobenzène)sulfonamide

Le produit attendu est obtenu selon le procédé décrit dans l'exemple 40 en remplaçant, au stade a, l'acide 6-aminocaproïque par l'acide 4-(aminométhyl)cyclohexanecarboxylique et le chlorure de 2-naphtylsulfonyle par le chlorure de 4-bromobenzènesulfonyle.
*Point de fusion : 192,194°C*

| *Microanalyse élémentaire : (x½ H*_{*2*}*O)* | | | |
|---|---|---|---|
| | *C* | *H* | *N* |
| *% calculé* | *50,48* | *5,05* | *8,83* |
| *% trouvé* | *50,38* | *5,15* | *8,69* |

### EXEMPLE 59 : N1-({4-[(2-Benzoylhydrazino)carbonyl]cyclohexyl}méthyl)-1-(4-bromobenzène)sulfonamide

Le produit attendu est obtenu selon le procédé décrit dans l'exemple 40 en remplaçant, au stade a, l'acide 6-aminocaproïque par l'acide 4-(aminométhyl)cyclohexanecarboxylique et le chlorure de 2-naphtylsulfonyle par le chlorure de 4-bromobenzènesulfonyle, et au stade b, la phénylhydrazine par la phénylhydrazide.
*Point de fusion : 251-253°C*

| *Microanalyse élémnetaire :* | | | |
|---|---|---|---|
| | *C* | *H* | *N* |
| *% calculé* | *51,02* | *4,89* | *8,50* |
| *% trouvé* | *50,47* | *4,92* | *8,34* |

### EXEMPLE 60 : N1-[(4-{[2-(4-Chlorophényl)hydrazino]cyclohexyl}carbonyl)méthyl]-1-(4-bromobenzène)sulfonamide

Le produit attendu est obtenu selon le procédé décrit dans l'exemple 40 en remplaçant, au stade a, l'acide 6-aminocaproïque par l'acide 4-(aminométhyl)cyclohexanecarboxylique et le chlorure de 2-naphtylsulfonyle par le chlorure de 4-bromobenzènesulfonyle, et au stade b, la phénylhydrazine par la 4-chlorophénylhydrazine.

| *Microanalyse élémentaire :* | | | |
|---|---|---|---|
| | *C* | *H* | *N* |
| *% calculé* | *47,96* | *4,63* | *8,39* |
| *% trouvé* | *47,99* | *4,63* | *8,29* |

### EXEMPLE 61 : N1-[(4-{[2-(4-Chlorobenzoyl)hydrazino]cyclohexyl}carbonyl)méthyl]-1-(4-bromobenzène)sulfonamide

Le produit attendu est obtenu selon le procédé décrit dans l'exemple 40 en remplaçant, au stade a, l'acide 6-aminocaproïque par l'acide 4-(aminométhyl)cyclohexanecarboxylique et le chlorure de 2-naphtylsulfonyle par le chlorure de 4-bromobenzènesulfonyle, et au stade b, la phénylhydrazine par la 4-chlorophénylhydrazide.

| *Microanalyse élémentaire :* | | | |
|---|---|---|---|
| | *C* | *H* | *N* |
| *% calculé* | *47,69* | *4,38* | *7,95* |
| *% trouvé* | *47,78* | *4,45* | *8,05* |

### EXEMPLE 62 : N'-Phényl-1-(phénylsulfonyl)perhydro-2-indolecarbohydrazide

### Stade a : Acide 1-(phénylsulfonyl)-2-perhydroindolecarboxylique

A une solution de 29,6 mmol (5 g) d'acide 2-perhydroindolecarboxylique dans 7,4 ml de NaOH 4M, refroidie à 0°C, sont ajoutés successivement 29,8 mmol (3,8 ml) de chlorure de benzène sulfonyle et 7,4 ml de soude 4M. La réaction est laissée à température ambiante sous agitation pendant 24h. Le milieu est ensuite acidifié à pH 2-3 et filtré. Le solide obtenu est lavé à l'éther pour conduire au produit attendu.

### Stade b : N'-Phényl-1-(phénylsulfonyl)perhydro-2-indolecarbohydrazide

Le produit attendu est obtenu selon le procédé décrit au stade b de l'exemple 1 en utilisant comme produit de départ le composé décrit au stade précédent.

| *Microanalyse élémentaire :* | | | |
|---|---|---|---|
| | *C* | *H* | *N* |
| *% calculé* | *63,16* | *6,27* | *10,53* |
| *% trouvé* | *63,37* | *6,43* | *10,38* |

### EXEMPLE 63 : N'-(2-indolylcarbonyl)-1-(phénylsulfonyl)perhydro-2-indolecarbohydrazide

Le produit attendu est obtenu selon le procédé décrit dans l'exemple 62 en remplaçant, au stade b, la phénylhydrazine par la 2-indolecarbohydrazide.
*Point de fusion : 134-137°C*

| *Microanalyse élémentaire :* | | | |
|---|---|---|---|
| | *C* | *H* | *N* |
| *% calculé* | *61,80* | *5,58* | *12,02* |
| *% trouvé* | *61,34* | *5,74* | *11,62* |

### EXEMPLE 64 : N'-Nicotinoyl-1-(phénylsulfonyl)perhydro-2-indolecarbohydrazide

Le produit attendu est obtenu selon le procédé décrit dans l'exemple 62 en remplaçant, au stade b, la phénylhydrazine par la 3-pyridinecarbohydrazide.

| *Microanalyse élémentaire :* | | | |
|---|---|---|---|
| | *C* | *H* | *N* |
| *% calculé* | *58,88* | *5,61* | *13,08* |
| *% trouvé* | *58,41* | *5,74* | *13,64* |

### EXEMPLE 65 : N2-({4-[(2-Phénylhydrazino)carbonyl]cyclohexyl}méthyl)-1-naphtalènesulfonamide

### Stade a : Acide 6-(1-naphtylsulfonyl)aminométhyl]cyclohexanecarboxylique

Le produit attendu est obtenu selon le procédé décrit dans l'exemple 40, stade a, en remplaçant l'acide 6-aminocaproïque par l'acide 4-(aminométhyl)cyclohexanecarboxylique et utilisant le chlorure de 1-naphtylsulfonyle.

### Stade b : N2-({4-[(2-Phénylhydrazino)carbonyl]cyclohexyl}méthyl)-1-naphtalènesulfonamide

Le produit attendu est obtenu selon le procédé décrit dans l'exemple 1, stade b, en utilisant comme produit de départ le composé décrit au stade précédent, et en remplaçant la phénylhydrazine par la phénylhydrazide.

| *Microanalyse élémentaire :* | | | |
|---|---|---|---|
| | *C* | *H* | *N* |
| *% calculé* | *64,44* | *5,80* | *9,02* |
| *% trouvé* | *64,59* | *5,96* | *8,76* |

### EXEMPLE 66 : N1-({4-[(2-Benzoylhydrazino)carbonyl]cyclohexyl}méthyl)-1-(4-isopropylbenzène)sulfonamide

Le produit attendu est obtenu selon le procédé décrit dans l'exemple 40, en remplaçant au stade a, l'acide 6-aminocaproïque par l'acide 4-(aminométhyl)cyclohexanecarboxylique, le chlorure de 2-naphtylsulfonyle par le chlorure de 4-isopropylbenzènesulfonyle, et au stade b, la phénylhydrazine par la phénylhydrazide.
*Point de fusion : 232-236°C*

| *Microanalyse élémentaire :* | | | |
|---|---|---|---|
| | *C* | *H* | *N* |
| *% calculé* | *62,46* | *6,77* | *9,10* |
| *% trouvé* | *62,32* | *7,20* | *9,18* |

### EXEMPLE 67 : N1-({4-[(2-Naphtoylhydrazino)carbonyl]cyclohexyl}méthyl)-1-(2-nitrobenzène)sulfonamide

Le produit attendu est obtenu selon le procédé décrit dans l'exemple 40, en remplaçant au stade a, l'acide 6-aminocaproïque par l'acide 4-(aminométhyl)cyclohexanecarboxylique, le chlorure de 2-naphtylsulfonyle par le chlorure de 2-nitrobenzènesulfonyle, et au stade b, la phénylhydrazine par la 2-naphtylhydrazide.
*Point de fusion : 263-264°C*

| *Microanalyse élémentaire :* | | | |
|---|---|---|---|
| | *C* | *H* | *N* |
| *% calculé* | *58,81* | *5,13* | *10,97* |
| *% trouvé* | *58,87* | *5,32* | *11,25* |

### EXEMPLE 68 : N1-({4-[(2-Benzoylhydrazino)carbonyl]cyclohexyl}méthyl)-1-(3-nitrobenzène)sulfonamide

Le produit attendu est obtenu selon le procédé décrit dans l'exemple 54, en remplaçant le chlorure de 2-nitrobenzènesulfonyle par le chlorure de 3-nitrobenzènesulfonyle.
*Point de fusion : 198-201°C*

| *Microanalyse élémentaire :* | | | |
|---|---|---|---|
| | *C* | *H* | *N* |
| *% calculé* | *51,69* | *4,92* | *11,48* |
| *% trouvé* | *51,58* | *4,83* | *11,61* |

### EXEMPLE 69 : N1-({4-[(2-Phénylhydrazino)carbonyl]cyclohexyl}méthyl)-1-(4-nitrobenzène)sulfonamide

Le produit attendu est obtenu selon le procédé décrit dans l'exemple 53, en remplaçant le chlorure de 2-nitrobenzènesulfonyle par le chlorure de 4-nitrobenzènesulfonyle.
*Point de fusion : 200-201°C*

| *Microanalyse élémentaire :* | | | |
|---|---|---|---|
| | *C* | *H* | *N* |
| *% calculé* | *54,36* | *5,44* | *12,68* |
| *% trouvé* | *53,99* | *5,33* | *12,46* |

### EXEMPLE 70 : N1-({4-[(2-Benzoylhydrazino)carbonyl]cyclohexyl}méthyl)-1-(4-nitrobenzène)sulfonamide

Le produit attendu est obtenu selon le procédé décrit dans l'exemple 54, en remplaçant le chlorure de 2-nitrobenzènesulfonyle par le chlorure de 4-nitrobenzènesulfonyle.
*Point de fusion : 262-264°C*

| *Microanalyse élémentaire :* | | | |
|---|---|---|---|
| | *C* | *H* | *N* |
| *% calculé* | *53,67* | *5,11* | *11,93* |
| *% trouvé* | *53,68* | *5,21* | *11,96* |

### EXEMPLE 71 : N1-[(4-{[2-(4-Chlorophényl)hydrazino]carbonyl}cyclohexyl)méthyl)-1-(4-nitrobenzène)sulfonamide

Le produit attendu est obtenu selon le procédé décrit dans l'exemple 40, en remplaçant au stade a, l'acide 6-aminocaproïque par l'acide 4-(aminométhyl)cyclohexanecarboxylique et le chlorure de 2-naphtylsulfonyle par le chlorure de 4-nitrobenzènesulfonyle, et au stade b, la phénylhydrazine par la 4-chlorophénylhydrazine.
*Point de fusion : 211-213°C*

| *Microanalyse élémentaire :* | | | |
|---|---|---|---|
| | *C* | *H* | *N* |
| *% calculé* | *51,45* | *4,96* | *12,00* |
| *% trouvé* | *51,52* | *5,12* | *11,81* |

### EXEMPLE 72 : N1-[(4-{[2-(4-Chlorobenzoyl)hydrazino]carbonyl}cyclohexyl)méthyl]-1-(4-nitrobenzène)sulfonamide

Le produit attendu est obtenu selon le procédé décrit dans l'exemple 40, en remplaçant au stade a, l'acide 6-aminocaproïque par l'acide 4-(aminométhyl)cyclohexanecarboxylique et le chlorure de 2-naphtylsulfonyle par le chlorure de 4-nitrobenzènesulfonyle, et au stade b, la phénylhydrazine par la 4-chlorophénylhydrazide.
*Point de fusion : 272-273°C*

| *Microanalyse élémentaire :* | | | |
|---|---|---|---|
| | *C* | *H* | *N* |
| *% calculé* | *50,91* | *4,65* | *11,31* |
| *% trouvé* | *50,89* | *4,76* | *11,29* |

### EXEMPLE 73 : N1-({4-[(2-(4-Phénylhydrazino)carbonyl]cyclohexyl}méthyl)-1-(2- bromobenzène)sulfonamide

Le produit attendu est obtenu selon le procédé décrit dans l'exemple 40, en remplaçant au stade a, l'acide 6-aminocaproïque par l'acide 4-(aminométhyl)cyclohexanecarboxylique et le chlorure de 2-naphtylsulfonyle par le chlorure de 2-bromobenzènesulfonyle.
*Point de fusion : 165-167°C*

| *Microanalyse élémentaire :* | | | |
|---|---|---|---|
| | *C* | *H* | *N* |
| *% calculé* | *51,46* | *5,14* | *9,01* |
| *% trouvé* | *51,54* | *5,29* | *9,03* |

### EXEMPLE 74 : N1-({4-[(2-Benzoylhydrazino)carbonyl]cyclohexyl}méthyl)-1-(2-bromobenzène)sulfonamide

Le produit attendu est obtenu selon le procédé décrit dans l'exemple 40, en remplaçant au stade a, l'acide 6-aminocaproïque par l'acide 4-(aminométhyl)cyclohexanecarboxylique et le chlorure de 2-naphtylsulfonyle par le chlorure de 2-bromobenzènesulfonyle, et au stade b, la phénylhydrazine par la phénylhydrazide.
*Point de fusion : 231-232°C*

| *Microanalyse élémentaire :* | | | |
|---|---|---|---|
| | *C* | *H* | *N* |
| *% calculé* | *50,97* | *4,85* | *8,49* |
| *% trouvé* | *51,11* | *4,98* | *8,51* |

### EXEMPLE 75 : N1-[(4-{[(2-(4-Chlorophényl)hydrazino]carbonyl}cyclohexyl) méthyl]-1-(2-bromobenzène)sulfonamide

Le produit attendu est obtenu selon le procédé décrit dans l'exemple 40, en remplaçant au stade a, l'acide 6-aminocaproïque par l'acide 4-(aminométhyl)cyclohexanecarboxylique et le chlorure de 2-naphtylsulfonyle par le chlorure de 2-bromobenzènesulfonyle, et au stade b, la phénylhydrazine par la 4-chlorophénylhydrazine.

| *Microanalyse élémentaire :* | | | |
|---|---|---|---|
| | *C* | *H* | *N* |
| *% calculé* | *47,92* | *4,59* | *8,39* |
| *% trouvé* | *48,15* | *4,73* | *8.37* |

### EXEMPLE 76 : N1-[(4-{[2-(4-Chlorophényl)hydrazino]cyclohexyl}carbonyl)méthyl]-1-(2-bromobenzène)sulfonamide

Le produit attendu est obtenu selon le procédé décrit dans l'exemple 40, en remplaçant au stade a, l'acide 6-aminocaproïque par l'acide 4-(aminométhyl)cyclohexanecarboxylique et le chlorure de 2-naphtylsulfonyle par le chlorure de 2-bromobenzènesulfonyle, et au stade b, la phénylhydrazine par la 4-chlorophénylhydrazide.
*Point de fusion : 263-264°C*

| *Microanalyse élémentaire :* | | | |
|---|---|---|---|
| | *C* | *H* | *N* |
| *% calculé* | *47,65* | *4,35* | *7,94* |
| *% trouvé* | *47,65* | *4,44* | *7,87* |

### EXEMPLE 77 : N1-({4-[(2-Benzoylhydrazino)carbonyl]cyclohexyl}méthyl)-1-(4-chlorobenzène)sulfonamide

Le produit attendu est obtenu selon le procédé décrit dans l'exemple 40, en remplaçant au stade a, l'acide 6-aminocaproïque par l'acide 4-(aminométhyl)cyclohexanecarboxylique et le chlorure de 2-naphtylsulfonyle par le chlorure de 4-chlorobenzènesulfonyle, et au stade b, la phénylhydrazine par la phénylhydrazide.
*Point de fusion : 254-256°C*

| *Microanalyse élémentaire :* | | | |
|---|---|---|---|
| | *C* | *H* | *N* |
| *% calculé* | *56,01* | *5,33* | *9,33* |
| *% trouvé* | *56,00* | *5,22* | *9,24* |

### EXEMPLE 78 : N1-({4-[(2-Benzoylhydrazino)carbonyl]cyclohexyl}méthyl)-1-(3,4-dichlorobenzène)sulfonamide

Le produit attendu est obtenu selon le procédé décrit dans l'exemple 40, en remplaçant au stade a, l'acide 6-aminocaproïque par l'acide 4-(aminométhyl)cyclohexanecarboxylique et le chlorure de 2-naphtylsulfonyle par le chlorure de 3,4-dichlorobenzènesulfonyle, et au stade b, la phénylhydrazine par la phénylhydrazide.
*Point de fusion : 251-252°C*

| *Microanalyse élémentaire :* | | | |
|---|---|---|---|
| | *C* | *H* | *N* |
| *% calculé* | *52,07* | *4,75* | *8,67* |
| *% trouvé* | *51,91* | *5,32* | *8,71* |

### EXEMPLE 79 : N1-({4-((2-Phénylhydrazino)carbonyl]cyclohexyl}méthyl)-1-(4-fluorobenzène)sulfonamide

Le produit attendu est obtenu selon le procédé décrit dans l'exemple 40, en remplaçant au stade a, l'acide 6-aminocaproïque par l'acide 4-(aminométhyl)cyclohexanecarboxylique et le chlorure de 2-naphtylsulfonyle par le chlorure de 4-fluorobenzènesulfonyle.
*Point de fusion : 192-193°C*

| *Microanalyse élémentaire :* | | | |
|---|---|---|---|
| | *C* | *H* | *N* |
| *% calculé* | *59,19* | *5,92* | *10,36* |
| *% trouvé* | *59,03* | *5,98* | *10,23* |

### EXEMPLE 80 : N1-({4-([(2-Benzoylhydrazino)carbonyl]cyclohexyl}méthyl)-1-(4-fluorobenzène)sulfonamide

Le produit attendu est obtenu selon le procédé décrit dans l'exemple 40, en remplaçant au stade a, l'acide 6-aminocaproïque par l'acide 4-(aminométhyl)cyclohexanecarboxylique et le chlorure de 2-naphtylsulfonyle par le chlorure de 4-fluorobenzènesulfonyle, et au stade b, la phénylhydrazine par la phénylhydrazide.
*Point de fusion : 264-265°C*

| *Microanalyse élémentaire :* | | | |
|---|---|---|---|
| | *C* | *H* | *N* |
| *% calculé* | *58,13* | *5,54* | *9,69* |
| *% trouvé* | *58,04* | *5,60* | *9,71* |

### EXEMPLE 81 : N1-[(4-{[2-(4-Chlorophényl)hydrazino]carbonyl}cyclohexyl)méthyl]-1-(4-fluorobenzène)sulfonamide

Le produit attendu est obtenu selon le procédé décrit dans l'exemple 40, en remplaçant au stade a, l'acide 6-aminocaproïque par l'acide 4-(aminométhyl)cyclohexanecarboxylique et le chlorure de 2-naphtylsulfonyle par le chlorure de 4-fluorobenzènesulfonyle, et au stade b, la phénylhydrazine par la 4-chlorophénylhydrazine.
*Point de fusion : 216-217°C*

| *Microanalyse élémentaire :* | | | |
|---|---|---|---|
| | *C* | *H* | *N* |
| *% calculé* | *54,31* | *5,20* | *9,50* |
| *% trouvé* | *54,39* | *5,22* | *9,45* |

### EXEMPLE 82 : N1-[(4-{[2-(4-Chlorobenzoyl)hydrazino]carbonyl}cyclohexyl) méthyl]-1-(4-fluorobenzène)sulfonamide

Le produit attendu est obtenu selon le procédé décrit dans l'exemple 40, en remplaçant au stade a, l'acide 6-aminocaproïque par l'acide 4-(aminométhyl)cyclohexanecarboxylique et le chlorure de 2-naphtylsulfonyle par le chlorure de 4-fluorobenzènesulfonyle, et au stade b, la phénylhydrazine par la 4-chlorophénylhydrazide.
*Point de fusion : 257-258°C*

| *Microanalyse élémentaire :* | | | |
|---|---|---|---|
| | *C* | *H* | *N* |
| *% calculé* | *53,85* | *4,92* | *8,98* |
| *% trouvé* | *53,90* | *5,00* | *8,89* |

### EXEMPLE 83 : N1-({4-[(2-Benzoylhydrazino)carbonyl]cyclohexyl}méthyl)-1-(4-méthylbenzène)sulfonamide

Le produit attendu est obtenu selon le procédé décrit dans l'exemple 40, en remplaçant au stade a, l'acide 6-aminocaproïque par l'acide 4-(aminométhyl)cyclohexanecarboxylique et le chlorure de 2-naphtylsulfonyle par le chlorure de 4-méthylbenzènesulfonyle, et au stade b, la phénylhydrazine par la phénylhydrazide.

| *Microanalyse élémentaire :* | | | |
|---|---|---|---|
| | *C* | *H* | *N* |
| *% calculé* | *61,52* | *6,34* | *9,78* |
| *% trouvé* | *61,71* | *6,32* | *9,67* |

### EXEMPLE 84 : N1-({4-[(2-Benzoylhydrazino)carbonyl]cyclohexyl}méthyl)-1-(4-méthoxybenzène)sulfonamide

Le produit attendu est obtenu selon le procédé décrit dans l'exemple 40, en remplaçant au stade a, l'acide 6-aminocaproïque par l'acide 4-(aminométhyl)cyclohexanecarboxylique et le chlorure de 2-naphtylsulfonyle par le chlorure de 4-méthoxybenzènesulfonyle, et au stade b, la phénylhydrazine par la phénylhydrazide.

| *Microanalyse élémentaire :* | | | |
|---|---|---|---|
| | *C* | *H* | *N* |
| *% calculé* | *59,25* | *6,06* | *9,43* |
| *% trouvé* | *59,25* | *6,26* | *9,49* |

### EXEMPLE 85 : N1-({4-[(2-Benzoylhydrazino]carbonyl]cyclohexyl}méthyl)-1-(3,4-diméthoxybenzène)sulfonamide

Le produit attendu est obtenu selon le procédé décrit dans l'exemple 40, en remplaçant au stade a, l'acide 6-aminocaproïque par l'acide 4-(aminométhyl)cyclohexanecarboxylique et le chlorure de 2-naphtylsulfonyle par le chlorure de 3,4-diméthoxybenzènesulfonyle, et au stade b, la phénylhydrazine par la phénylhydrazide.
*Point de fusion : 244-245°C*

| *Microanalyse élémentaire :* | | | |
|---|---|---|---|
| | *C* | *H* | *N* |
| *% calculé* | *57,02* | *5,99* | *8,68* |
| *% trouvé* | *56,91* | *6,33* | *8,84* |

### EXEMPLE 86 : N1-({4-[(2-Benzoylhydrazino)carbonyl]cyclohexyl}méthyl)-1-(4-acétylaminobenzène)sulfonamide

Le produit attendu est obtenu selon le procédé décrit dans l'exemple 40, en remplaçant au stade a, l'acide 6-aminocaproïque par l'acide 4-(aminométhyl)cyclohexanecarboxylique et le chlorure de 2-naphtylsulfonyle par le chlorure de 4-(N-acétylamino)benzènesulfonyle, et au stade b, la phénylhydrazine par la phénylhydrazide.

| *Microanalyse élémentaire :* | | | |
|---|---|---|---|
| | *C* | *H* | *N* |
| *% calculé* | *58,46* | *5,97* | *11,86* |
| *% trouvé* | *58,29* | *6,04* | *11,73* |

### EXEMPLE 87 : N1-({4-[(2-Benzoylhydrazino)carbonyl]cyclohexyl}méthyl)-1-(2-méthylsulfonylbenzène)sulfonamide

Le produit attendu est obtenu selon le procédé décrit dans l'exemple 40, en remplaçant au stade a, l'acide 6-aminocaproïque par l'acide 4-(aminométhyl)cyclohexanecarboxylique et le chlorure de 2-naphtylsulfonyle par le chlorure de 2-(méthylsulfonyl)benzènesulfonyle, et au stade b, la phénylhydrazine par la phénylhydrazide.
*Point de fusion : 193-194°C*

| *Microanalyse élémentaire :* | | | |
|---|---|---|---|
| | *C* | *H* | *N* |
| *% calculé* | *53,55* | *5,48* | *8,52* |
| *% trouvé* | *53,17* | *5,74* | *8,49* |

### EXEMPLE 88 : N1-({4-[(2-Benzoylhydrazino)carbonyl]cyclohexyl}méthyl)-1-(β-styrène)sulfonamide

Le produit attendu est obtenu selon le procédé décrit dans l'exemple 40, en remplaçant au stade a, l'acide 6-aminocaproïque par l'acide 4-(aminométhyl)cyclohexanecarboxylique et le chlorure de 2-naphtylsulfonyle par le chlorure de (β-styrène)sulfonyle, et au stade b, la phénylhydrazine par la phénylhydrazide.
*Point de fusion : 208-209°C*

| *Microanalyse élémentaire :* | | | |
|---|---|---|---|
| | *C* | *H* | *N* |
| *% calculé* | *60,13* | *6,32* | *9,15* |
| *% trouvé* | *60,06* | *6,45* | *9,05* |

### EXEMPLE 89 : N1-({4-[(2-Phénylhydrazino)carbonyl]cyclohexyl}méthyl)-1-(2-thiényl)sulfonamide

Le produit attendu est obtenu selon le procédé décrit dans l'exemple 40, en remplaçant au stade a, l'acide 6-aminocaproïque par l'acide 4-(aminométhyl)cyclohexanecarboxylique et le chlorure de 2-naphtylsulfonyle par le chlorure de (2-thiényl)sulfonyle.
*Point de fusion : 218-221°C*

| *Microanalyse élémentaire :* | | | |
|---|---|---|---|
| | *C* | *H* | *N* |
| *% calculé* | *54,89* | *5,84* | *10,67* |
| *% trouvé* | *54,50* | *6,05* | *10,82* |

### EXEMPLE 90 : N1-({4-[(2-Benzoylhydrazino)carbonyl]cyclohexyl}méthyl)-1-(2-thiényl)sulfonamide

Le produit attendu est obtenu selon le procédé décrit dans l'exemple 40, en remplaçant au stade a, l'acide 6-aminocaproïque par l'acide 4-(aminométhyl)cyclohexanecarboxylique et le chlorure de 2-naphtylsulfonyle par le chlorure de (2-thiényl)sulfonyle, et au stade b, la phénylhydrazine par la phénylhydrazide.
*Point de fusion : 221-222°C*

| *Microanalyse élémentaire :* | | | |
|---|---|---|---|
| | *C* | *H* | *N* |
| *% calculé* | *52,96* | *5,34* | *9,76* |
| *% trouvé* | *53,14* | *5,50* | *9,82* |

### EXEMPLE 91 : N1-[(4-{[2-(4-Chlorophényl)hydrazino]carbonyl}cyclohexyl)méthyl]-1-(2-thiényl)sulfonamide

Le produit attendu est obtenu selon le procédé décrit dans l'exemple 40, en remplaçant au stade a, l'acide 6-aminocaproïque par l'acide 4-(aminométhyl)cyclohexanecarboxylique et le chlorure de 2-naphtylsulfonyle par le chlorure de (2-thiényl)sulfonyle, et au stade b, la phénylhydrazine par la 4-chlorophénylhydrazine.
*Point de fusion : 193-194°C*

| *Microanalyse élémentaire :* | | | |
|---|---|---|---|
| | *C* | *H* | *N* |
| *% calculé* | *50,47* | *5,14* | *9,81* |
| *% trouvé* | *50,50* | *5,27* | *9,73* |

### EXEMPLE 92 : N1-[(4-{[(2-(4-Chlorobenzoyl)hydrazino]carbonyl}cyclohexyl) méthyl]-1-(2-thiényl)sulfonamide

Le produit attendu est obtenu selon le procédé décrit dans l'exemple 40, en remplaçant au stade a, l'acide 6-aminocaproïque par l'acide 4-(aminométhyl)cyclohexanecarboxylique et le chlorure de 2-naphtylsulfonyle par le chlorure de (2-thiényl)sulfonyle, et au stade b, la phénylhydrazine par la 4-chlorophénylhydrazide.
*Point de fusion : 237-239°C*

| *Microanalyse élémentaire :* | | | |
|---|---|---|---|
| | *C* | *H* | *N* |
| *% calculé* | *50,00* | *4,82* | *9,21* |
| *% trouvé* | *49,93* | *4,97* | *9,37* |

### EXEMPLE 93 : N1-(6-Oxo-6-(2-phénylhydrazino)hexyl]-1-benzènesulfonamide

Le produit attendu est obtenu selon le procédé décrit dans l'exemple 40, en remplaçant le chlorure de 2-naphtylsulfonyle par le chlorure de benzènesulfonyle.
*Point de fusion : 123-124°C*

| *Microanalyse élémentaire :* | | | |
|---|---|---|---|
| | *C* | *H* | *N* |
| *% calculé* | *59,81* | *6,41* | *11,62* |
| *% trouvé* | *59,92* | *6,58* | *11,52* |

### EXEMPLE 94 : N1-[6-(2-Benzoylhydrazino)-6-oxohexyl]-1-phénylsulfonamide

Le produit attendu est obtenu selon le procédé décrit dans l'exemple 40, en remplaçant le chlorure de 2-naphtylsulfonyle par le chlorure de benzènesulfonyle, et la phénylhydrazine par la phénylhdrazide.
*Point de fusion : 150°C*

| *Microanalyse élémentaire :* | | | |
|---|---|---|---|
| | *C* | *H* | *N* |
| *% calculé* | *58,59* | *5,95* | *10,79* |
| *% trouvé* | *58,52* | *6,10* | *10,90* |

### EXEMPLE 95 : N-[1-(4-Méthoxybenzyl)-2-oxo-2-(2-phénylsulfonylhydrazino)éthyl] carbamate de benzyle

Le produit attendu est obtenu selon le procédé décrit dans l'exemple 1, en remplaçant au stade a, la phénylalanine par la O-méthyltyrosine et au stade b, la phénylhydrazine par la phénylsulfonylhydrazine.

| *Microanalyse élémentaire :* | | | |
|---|---|---|---|
| | *C* | *H* | *N* |
| *% calculé* | *59,63* | *5,18* | *8,70* |
| *% trouvé* | *60,03* | *5,41* | *8,65* |

### EXEMPLE 96 : N1-[1-(4-Méthoxybenzyl)-2-oxo-2-(2-phénylsulfonylhydrazino)éthyl] -2-phénoxyacétamide

Le produit attendu est obtenu selon le procédé décrit dans l'exemple 5, en remplaçant au stade a, la phénylalanine par la O-méthyltyrosine et au stade b, la phénylhydrazine par la phénylsulfonylhydrazine.

| *Microanalyse élémentaire :* | | | |
|---|---|---|---|
| | *C* | *H* | *N* |
| *% calculé* | *59,63* | *5,18* | *8,70* |
| *% trouvé* | *59,52* | *5,06* | *8,59* |

### EXEMPLE 97 : N1-[1-Benzyl-2-oxo-2-(2-phénylsulfonylhydrazino)éthyl)-1-phénylsulfonamide

Le produit attendu est obtenu selon le procédé décrit dans l'exemple 10, en remplaçant au stade a, le chlorure de 2-naphtylsulfonyle par le chlorure de benzènesulfonyle, et au stade b la phénylhydrazine par la phénylsulfonylhydrazine.
*Point de fusion : 182-183°C*

| *Microanalyse élémentaire :* | | | |
|---|---|---|---|
| | *C* | *H* | *N* |
| *% calculé* | *54,90* | *4,58* | *9,15* |
| *% trouvé* | *54,70* | *4,79* | *8,91* |

### EXEMPLE 98 : N2-(1-Benzyl-2-oxo-2-(2-phénylsulfonylhydrazino)éthyl]-2-naphtylamide

Le produit attendu est obtenu selon le procédé décrit dans l'exemple 1, en remplaçant au stade a, le chloroformiate de benzyle par le chlorure de naphtoyle, et au stade b la phénylhydrazine par la phénylsulfonylhydrazine.

| *Microanalyse élémentaire :* | | | |
|---|---|---|---|
| | *C* | *H* | *N* |
| *% calculé* | *64,41* | *4,97* | *8,35* |
| *% trouvé* | *63,95* | *5,01* | *8,29* |

### EXEMPLE 99 : 1-[(4-Chlorophényl)sulfonyl]-N'-phénylperhydro-2-indolecarbohydrazide

Le produit attendu est obtenu selon le procédé décrit dans l'exemple 62, en remplaçant au stade a, le chlorure de benzènesulfonyle par le chlorure de 4-chlorobenzènesulfonyle.

| *Microanalyse élémentaire :* | | | |
|---|---|---|---|
| | *C* | *H* | *N* |
| *% calculé* | *58,13* | *5,54* | *9,68* |
| *% trouvé* | *58,66* | *5,63* | *9,18* |

### EXEMPLE 100 : 1-[(4-Chlorophényl)sulfonyl]-N'-nicotinoyl-perhydro-2- indolecarbohydrazide

Le produit attendu est obtenu selon le procédé décrit dans l'exemple 62, en remplaçant au stade a, le chlorure de benzènesulfonyle par le chlorure de 4-chlorobenzènesulfonyle, et au stade b la phénylhydrazine par la 3-pyridinecarbohydrazide.

| *Microanalyse élémentaire :* | | | |
|---|---|---|---|
| | *C* | *H* | *N* |
| *% calculé* | *54,49* | *4,97* | *12,11* |
| *% trouvé* | *54,54* | *5,21* | *12,32* |

### EXEMPLE 101 : 1-[(2-Nitrophényl)sulfonyl]-N'-phénylperhydro-2-indolecarbohydrazide

Le produit attendu est obtenu selon le procédé décrit dans l'exemple 62, en remplaçant au stade a, le chlorure de benzènesulfonyle par le chlorure de 2-nitrobenzènesulfonyle.
*Point de fusion : 86-89°C*

| *Microanalyse élémentaire :* | | | |
|---|---|---|---|
| | *C* | *H* | *N* |
| *% calculé* | *56,88* | *5,19* | *12,64* |
| *% trouvé* | *56,78* | *5,49* | *12,26* |

### EXEMPLE 102 : N'-Benzoyl-1-[(2-Nitrophényl)sulfonyl]-perhydro-2-indolecarbohydrazide

Le produit attendu est obtenu selon le procédé décrit dans l'exemple 62, en remplaçant au stade a, le chlorure de benzènesulfonyle par le chlorure de 2-nitrobenzènesulfonyle, et au stade b, la phénylhydrazine par la phénylhydrazide.
*Point de fusion : 109-113°C*

| *Microanalyse élémentaire :* | | | |
|---|---|---|---|
| | *C* | *H* | *N* |
| *% calculé* | *55,93* | *5,08* | *11,86* |
| *% trouvé* | *56,21* | *5,24* | *12,05* |

### EXEMPLE 103 : 1-[(4-Chlorophényl)sulfonyl]-N'-phényl-2-pyrrolidine-carbohydrazide

Le produit attendu est obtenu selon le procédé décrit dans l'exemple 62, en remplaçant au stade a, l'acide perhydroindolecarboxylique par la proline et le chlorure de benzènesulfonyle par le chlorure de 4-chlorobenzènesulfonyle.
*Point de fusion : 133-135°C*

| *Microanalyse élémentaire :* | | | |
|---|---|---|---|
| | *C* | *H* | *N* |
| *% calculé* | *53,75* | *4,74* | *11,07* |
| *% trouvé* | *53,94* | *4,89* | *10,81* |

### EXEMPLE 104 : N'-Benzoyl-1-[(4-chlorophényl)sulfonyl]-2-pyrrolidine-carbohydrazide

Le produit attendu est obtenu selon le procédé décrit dans l'exemple 62, en remplaçant au stade a, l'acide perhydroindolecarboxylique par la proline et le chlorure de benzènesulfonyle par le chlorure de 4-chlorobenzènesulfonyle, et au stade b, la phénylhydrazine par la phénylhydrazide.
*Point de fusion : 171-172°C*

| *Microanalyse élémenlaire :* | | | |
|---|---|---|---|
| | *C* | *H* | *N* |
| *% calculé* | *53,01* | *4,42* | *10,31* |
| *% trouvé* | *53,26* | *4,53* | *10,36* |

### EXEMPLE 105 : 1-[(4-Chlorophényl)sulfonyl]-N'-nicotinoyl-2-pyrrolidine-carbohydrazide

Le produit attendu est obtenu selon le procédé décrit dans l'exemple 62, en remplaçant au stade a, l'acide perhydroindolecarboxylique par la proline et le chlorure de benzènesulfonyle par le chlorure de 4-chlorobenzènesulfonyle, et au stade b, la phénylhydrazine par la 3-pyridinecarbohydrazide.
*Point de fusion : 107-108°C*

| *Microanalyse élémentaire :* | | | |
|---|---|---|---|
| | *C* | *H* | *N* |
| *% calculé* | *49,94* | *4,16* | *13,71* |
| *% trouvé* | *50,51* | *4,40* | *13,47* |

### EXEMPLE 106 : 1-[(4-Chlorophényl)sulfonyl]-N'-naphtyl-2-pyrrolidine-carbohydrazide

Le produit attendu est obtenu selon le procédé décrit dans l'exemple 62, en remplaçant au stade a, l'acide perhydroindolecarboxylique par la proline et la phénylhydrazine par la naphtylhydrazine.
*Point de fusion : 178-180°C*

| *Microanalyse élémentaire :* | | | |
|---|---|---|---|
| | *C* | *H* | *N* |
| *% calculé* | *60,80* | *4,58* | *6,76* |
| *% trouvé* | *60,78* | *4,73* | *6,79* |

### EXEMPLE 107 : N-({4-[(2-Benzoylhydrazino)carbonyl]cyclohexyl}méthyl)-5-(diméthylamino)-1-naphtalènesulfonamide

Le produit attendu est obtenu selon le procédé décrit dans l'exemple 40, en remplaçant au stade a, l'acide 6-aminocaproïque par l'acide 4-(aminométhyl)cyclohexanecarboxylique et le chlorure de naphtylsulfonyle par le chlorure de 5-diméthylamino-1-naphtalènesulfonyle, et au stade b la phénylhydrazine par la phénylhydrazide.
*Point de fusion : 201-203°C*

| *Microanalyse élémentaire :* | | | |
|---|---|---|---|
| | *C* | *H* | *N* |
| *% calculé* | *63,78* | *6,30* | *11,02* |
| *% trouvé* | *63,31* | *6,02* | *10,92* |

### EXEMPLE 108 : 4-Bromo-N-({4-[(2-{[4-(trifluorométhyl)-2-pyrimidinyl]carbonyl} hydrazino)carbonyl]cyclohexyl}méthyl)benzènesulfonamide

Le produit attendu est obtenu selon le procédé décrit dans l'exemple 40, en remplaçant au stade a, l'acide 6-aminocaproïque par l'acide 4-(aminométhyl)cyclohexanecarboxylique et le chlorure de naphtylsulfonyle par le chlorure de 4-bromobenzènesulfonyle. et au stade b la phénylhydrazine par la 4-(trifluorométhyl)-2-pyrimidinecarbohydrazide.
*Point de fusion : 229-230°C*

| *Microanalyse élémentaire :* | | | |
|---|---|---|---|
| | *C* | *H* | *N* |
| *% calculé* | *41,16* | *4,15* | *12,64* |
| *% trouvé* | *41,06* | *4,03* | *12,75* |

### EXEMPLE 109 : 3,4-Diméthoxy-N-[(4-{[2-(3-pyridylcarbonyl)hydrazino]carbonyl} cyclohexyl)méthyl]benzènesulfonamide

Le produit attendu est obtenu selon le procédé décrit dans l'exemple 40, en remplaçant au stade a, l'acide 6-aminocaproïque par l'acide 4-(aminométhyl)cyclohexanecarboxylique et le chlorure de naphtylsulfonyle par le chlorure de 3,4-diméthoxybenzènesulfonyle, et au stade b la phénylhydrazine par la 3-pyridinecarbohydrazide.
*Point de fusion : 211-212°C*

| *Microanalyse élémentaire :* | | | |
|---|---|---|---|
| | *C* | *H* | *N* |
| *% calculé* | *55,46* | *5,88* | *11,76* |
| *% trouvé* | *55,43* | *5,75* | *11,86* |

### EXEMPLE 110 : N-({4-[(2-Benzoylhydrazino)carbonyl]cyclohexyl}méthyl)-5-quinolinesulfonamide

Le produit attendu est obtenu selon le procédé décrit dans l'exemple 40, en remplaçant au stade a, l'acide 6-aminocaproïque par l'acide 4-(aminométhyl)cyclohexanecarboxylique et le chlorure de naphtylsulfonyle par le chlorure de 5-diméthylamino-1-naphtalènesulfonyle, et au stade b la phénylhydrazine par la phénylhydrazide.
*Point de fusion : 215-216°C*

| *Microanalyse élémentaire :* | | | |
|---|---|---|---|
| | *C* | *H* | *N* |
| *% calculé* | *61,80* | *5,58* | *12,02* |
| *% trouvé* | *61,69* | *5,66* | *12,13* |

### EXEMPLE 111 : N-{4-[(2-Benzoylhydrazino)carbonyl]benzyl}benzènesulfonamide

Le produit attendu est obtenu selon le procédé décrit dans l'exemple 40, en remplaçant au stade a, l'acide 6-aminocaproïque par l'acide 4-(aminométhyl)benzoïque et le chlorure de 2-naphtylsulfonyle par le chlorure de benzènesulfonyle, et au stade b la phénylhydrazine par la phénylhydrazide.
*Point de fusion : 191-193°C*

| *Microanalyse élémentaire :* | | | |
|---|---|---|---|
| | *C* | *H* | *N* |
| *% calculé* | *61,54* | *4,64* | *10,26* |
| *% trouvé* | *61,57* | *4,81* | *10,22* |

### EXEMPLE 112 : N2-{4-[(2-Benzoylhydrazino)carbonyl]benzyl} naphtalènesulfonamide

Le produit attendu est obtenu selon le procédé décrit dans l'exemple 40, en remplaçant au stade a, l'acide 6-aminocaproïque par l'acide 4-(aminométhyl)benzoïque, et au stade b la phénylhydrazine par la phénylhydrazide.

| *Microanalyse élémentaire :* | | | |
|---|---|---|---|
| | *C* | *H* | *N* |
| *% calculé* | *65,29* | *4,57* | *9,14* |
| *% trouvé* | *65,69* | *4,70* | *8,87* |

### EXEMPLE 113 : N-{4-[(2-Benzoylhydrazino)carbonyl]benzyl}-2-nitrobenzènesulfonamide

Le produit attendu est obtenu selon le procédé décrit dans l'exemple 40, en remplaçant au stade a, l'acide 6-aminocaproïque par l'acide 4-(aminométhyl)benzoïque et le chlorure de 2-naphtylsulfonyle par le chlorure de 2-nitrobenzènesulfonyle. et au stade b la phénylhydrazine par la phénylhydrazide.

| *Microanalyse élémentaire :* | | | |
|---|---|---|---|
| | *C* | *H* | *N* |
| *% calculé* | *55,45* | *3,96* | *12,32* |
| *% trouvé* | *55,36* | *4,11* | *12,56* |

### EXEMPLE 114 : N-{4-[(2-Benzoylhydrazino)carbonyl]benzyl}-4- bromobenzènesulfonamide

Le produit attendu est obtenu selon le procédé décrit dans l'exemple 40, en remplaçant au stade a, l'acide 6-aminocaproïque par l'acide 4-(aminométhyl)benzoïque et le chlorure de 2-naphtylsulfonyle par le chlorure de 4-bromobenzènesulfonyle, et au stade b la phénylhydrazine par la phénylhydrazide.
*Point de fusion : 208-210°C*

| *Microanalyse élémentaire :* | | | |
|---|---|---|---|
| | *C* | *H* | *N* |
| *% calculé* | *50,71* | *3,82* | *8,45* |
| *% trouvé* | *50,85* | *3,61* | *8,42* |

### ETUDE PHARMACOLOGIQUE

### EXEMPLE A : Mesure de l'affinité in vitro aux récepteurs du NPY

La capacité des composés de l'invention à se lier aux récepteurs du NPY a été mesurée sur différentes lignées cellulaires exprimant chacune un des sous-types réceptoriels étudiés. Des expériences de liaison compétitive ont été réalisées en utilisant comme radioligand le peptide [¹²⁵I]-PYY à des concentrations variant de 15 à 65 pM. La fraction non spécifique est mesurée en présence d'une concentration de 1 µM de NPY. Les cellules sont incubées pendant une période variant de 1 à 2 heures suivant les lignées, et la radioactivité est recueillie après filtration sur filtre GF/C traité par du PEI à 0,1 %, avant d'être mesurée.

### Résultats :

Les résultats sont exprimés en IC₅₀. Il apparaît que les composés de l'invention sont capables de déplacer de façon importante le ligand de référence : les IC₅₀ varient de quelques nanomoles à quelques centaines de nanomoles.

A titre d'exemple, le composé de l'exemple 44 possède une IC₅₀ de 14,5 nM pour le récepteur Y₅.

### EXEMPLE B : Mesure de l'effet sur la prise alimentaire et l'évolution pondérale chez la souris obèse

Les produits de l'invention ont été traités in vivo chez la souris obèse ob/ob, afin d'évaluer leur influence sur la prise alimentaire et l'évolution pondérale. Les animaux utilisés sont des souris C57B1/6J ob/ob femelles, âgées de 13 à 18 semaines. Ils sont répartis en lots de 4 animaux par cage équipées d'un sol grillagé et d'un accès libre à la nourriture.

Avant les expériences, les animaux sont conditionnés pendant une période variant de 2 à 3 semaines, jusqu'à une consommation alimentaire stabilisée. Le synoptique des expériences est le suivant :
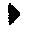 J - 14 à J - 7 : conditionnement
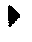 J - 7 à J - 3 : mesure de la prise alimentaire basale
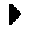 J0 à J + 3 : animaux traités 2 fois par jour, les lots contrôle recevant le véhicule
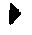 J0 à J + 4 : mesure quotidienne de la prise alimentaire et de poids corporel

Les produits testés sont mis en solution extemporanément dans l'eau, le chlorure de sodium à 0,9 %, le propylène glycol ou le diméthylsulfoxide en fonction de leur solubilité, et sont administrés par voie intrapéritonéale (IP), à un volume de 2,5 ml/kg.

Les paramètres mesurés sont le poids des mangeoires contenant la nourriture et le poids corporel.

### Résultats :

Les résultats sont exprimés en :
- pourcentage de variation de la prise alimentaire sous traitement par rapport à la prise alimentaire basale ;
- pourcentage de variation du poids corporel entre le premier et le dernier jour de traitement.

A titre d'exemple, les résultats obtenus avec le composé de l'exemple 44 sont les suivants :

| Produit | Dose (mg/kg) | Prise alimentaire % variation (J1) | | Poids corporel % variation (J4/J0) |
|---|---|---|---|---|
| | | Contrôle | Traités | |
| Exemple 44 | 5 | - 25,3 | - 75,2 | - 6,0 |

### EXEMPLE C : Etude de la toxicité aiguë

La toxicité aiguë a été appréciée après administration orale à des lots de 8 souris (26 ± 6 grammes) de doses croissantes de produit à étudier. Les animaux ont été observés à intervalles réguliers au cours de la première journée et quotidiennement pendant les deux semaines suivant le traitement.
Il apparaît que les composés de l'invention sont très peu toxiques.

### EXEMPLE D : Composition pharmaceutique

| Formule de préparation pour 1000 comprimés dosés à 10 mg | |
|---|---|
| Composé de l'exemple 44 | 10 g |
| Hydroxypropylcellulose | 2 g |
| Amidon de blé | 10 g |
| Lactose | 100 g |
| Stéarate de magnésium | 3 g |
| Talc | 3 g |

## Revendications

1. Composés de formule (I) :
R - NH - A - CO - NH - NH - (W)ₙ - Z (I)
dans laquelle :
◆ n vaut 0 ou 1,
◆ W représente un groupement -CO- ou un groupement S(O)ᵣ dans lequel r vaut 0, 1 ou 2,
◆ Z représente un groupement choisi parmi aryle éventuellement substitué, arylalkyle éventuellement substitué, hétéroaryle éventuellement substitué et hétéroarylalkyle éventuellement substitué,
◆ R représente un groupement choisi parmi :
Z₁-T-CO-
Z₁-O-T-CO-
Z₁-T-O-CO-
Z₁-T-S(O)_{q}-
dans lesquels :
- Z₁ représente un groupement aryle éventuellement substitué, arylalkyle éventuellement substitué, hétéroaryle éventuellement substitué ou hétéroarylalkyle éventuellement substitué,
- T représente une liaison σ ou un groupement alkylène, alkénylène ou alkynylène,
- q représente un entier égal à 0, 1 ou 2,
◆ A représente un groupement alkylène linéaire ou ramifié de 3 à 8 atomes de carbone, alkénylène linéaire ou ramifié de 3 à 8 atomes de carbone, alkynylène linéaire ou ramifié de 3 à 8 atomes de carbone, alkylènecycloalkylène, cycloalkylènealkylène, alkylènecycloalkylènealkylène, alkylènearylène, arylènealkylène, alkylènearylènealkylène, un groupement 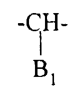 dans lequel B₁ représente un groupement aryle éventuellement substitué, arylalkyle éventuellement substitué, hétéroaryle éventuellement substitué, hétéroarylalkyle éventuellement substitué, ou bien A forme avec l'atome d'azote un groupement 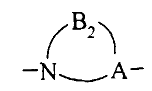 dans lequel B₂ représente un système mono ou bicyclique de 5 à 11 chaînons saturé ou insaturé comportant éventuellement de 1 à 3 hétéroatomes supplémentaires choisis parmi azote, oxygène et soufre,
à la condition que lorsque simultanément n vaut 0, A représente un groupement 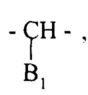 B₁ étant un groupement benzyle, et Z représente un groupement phényle éventuellement substitué, alors R est différent d'un groupement benzoyle,
leurs énantiomères, diastéréoisomères, ainsi que leurs sels d'addition à un acide ou à une base pharmaceutiquement acceptable,
étant entendu que,
le terme alkyle désigne un groupement linéaire ou ramifié de 1 à 6 atomes de carbone,
le terme alkylène désigne un radical bivalent, linéaire ou ramifié, contenant de 1 à 6 atomes de carbones, sauf précisions contraires,
le terme alkénylène désigne un radical bivalent linéaire ou ramifié, contenant de 2 à 6 atomes de carbones et 1 à 3 doubles liaisons, sauf précisions contraires,
le terme alkynylène désigne un radical bivalent, linéaire ou ramifié, contenant de 2 à 6 atomes de carbone et 1 à 3 triples liaisons, sauf précisions contraires,
le terme aryle désigne un groupement phényle, napthyle, dihydronapthyle, ou tétrahydronapthyle, le terme arylène désignant un groupement bivalent du même type,
le terme hétéroaryle désigne un groupement mono ou bicyclique insaturé ou partiellement insaturé, de 5 à 11 chaînons, contenant de 1 à 4 hétéroatomes choisis parmi azote, oxygène et soufre,
le terme alkylènecycloalkylène représente un groupement -A₁-A₂-, le terme cycloalkylènealkylène représente un groupement -A₂-A₁-, et le terme alkylènecycloalkylènealkylène représente un groupement -A₁-A₂-A₁, le terme alkylènearylène représente un groupement -A₁-A₃-, le terme arylènealkylène représente un groupement -A₃-A₁-, le terme alkylènearylènealkylène représente un groupement -A₃-A₁-A₃, dans lesquels A₁ est un groupement alkylène tel que défini précédemment, A₂ est un groupement cycloalkylène (C₄-C₈), et A₃ est un groupement arylène tel que défini précédemment,
l'expression éventuellement substitué affectée aux termes aryle, arylalkyle, hétéroaryle, hétéroarylalkyle, signifie que ces groupements sont substitués sur leur partie cyclique par 1 à 5 substituants, identiques ou différents, choisis parmi alkyle (C₁-C₆) linéaire ou ramifié, alkoxy (C₁-C₆) linéaire ou ramifié, halogène, hydroxy, perhalogénoalkyle (C₁-C₆) linéaire ou ramifié, nitro, acyle (C₁-C₆) linéaire ou ramifié, alkylsulfonyle (C₁-C₆) linéaire ou ramifié, et amino (éventuellement substitué par un ou deux groupements alkyle (C₁-C₆) linéaire ou ramifié, ou acyle (C₁-C₆) linéaire ou ramifié).

2. Composés de formule (I) selon la revendication 1 pour lesquels W représente un groupement -CO-, leurs énantiomères, diastéréoisomères, ainsi que leurs sels d'addition à un acide ou à une base pharmaceutiquement acceptable.

3. Composés de formule (I) selon la revendication 1 pour lesquels W représente un groupement -SO₂-, leurs énantiomères, diastéréoisomères, ainsi que leurs sels d'addition à un acide ou à une base pharmaceutiquement acceptable.

4. Composés de formule (1) selon la revendication 1 pour lesquels R représente un groupement Z₁-T-CO, T étant un groupement alkylène ou une liaison, leurs énantiomères, diastéréoisomères, ainsi que leurs sels d'addition à un acide ou à une base pharmaceutiquement acceptable.

5. Composés de formule (I) selon la revendication 1 pour lesquels R représente un groupement Z₁-O-T-CO, T étant un groupement alkylène ou une liaison, leurs énantiomères, diastéréoisomères, ainsi que leurs sels d'addition à un acide ou à une base pharmaceutiquement acceptable.

6. Composés de formule (I) selon la revendication 1 pour lesquels R représente un groupement Z₁-T-O-CO, T étant un groupement alkylène ou une liaison, leurs énantiomères, diastéréoisomères, ainsi que leurs sels d'addition à un acide ou à une base pharmaceutiquement acceptable.

7. Composés de formule (I) selon la revendication 1 pour lesquels R représente un groupement Z₁-T-S(O)_{q}-, T étant un groupement alkylène ou une liaison, leurs énantiomères, diastéréoisomères, ainsi que leurs sels d'addition à un acide ou à une base pharmaceutiquement acceptable.

8. Composés de formule (I) selon la revendication 1 pour lesquels Z représente un groupement aryle éventuellement substitué, ou hétéroaryle éventuellement substitué, leurs énantiomères, diastéréoisomères, ainsi que leurs sels d'addition à un acide ou à une base pharmaceutiquement acceptable.

9. Composés de formule (I) selon la revendication 1 pour lesquels A représente un groupement 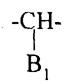 dans lequel B₁ est un groupement arylalkyle éventuellement substitué, leurs énantiomères, diastéréoisomères, ainsi que leurs sels d'addition à un acide ou à une base pharmaceutiquement acceptable.

10. Composés de formule (I) selon la revendication 1 pour lesquels A représente un groupement alkylènecycloalkylène, leurs énantiomères, diastéréoisomères, ainsi que leurs sels d'addition à un acide ou à une base pharmaceutiquement acceptable.

11. Composés de formule (I) selon la revendication 1 pour lesquels A représente un groupement alkylènearylène, leurs énantiomères, diastéréoisomères, ainsi que leurs sels d'addition à un acide ou à une base pharmaceutiquement acceptable.

12. Composés de formule (I) selon la revendication 1 pour lesquels A représente un groupement 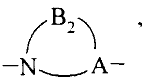 leurs énantiomères, diastéréoisomères, ainsi que leurs sels d'addition à un acide ou à une base pharmaceutiquement acceptable.

13. Composé de formule (I) selon la revendication 1 pour lesquels W représente un groupement -CO-, Z représente un groupement choisi parmi aryle éventuellement substitué, et hétéroaryle éventuellement substitué, R représente un groupement choisi parmi Z₁-T-CO-, Z₁-O-T-CO-, Z₁-T-O-CO-, Z₁-T-S(O)_{q}- dans lesquels Z₁ est un groupement aryle éventuellement substitué, ou hétéroaryle éventuellement substitué, T représente un groupement alkylène, et q vaut 2, et A représente un groupement alkylènecycloalkylène, un groupement 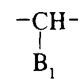 dans lequel B₁ est un groupement arylalkyle éventuellement substitué, ou bien A forme avec l'atome d'azote adjacent un groupement pyrrolidine, perhydroindole ou pipéridine, ainsi que ses énantiomères, diastéréoisomères, ainsi que ses sels d'addition à un acide ou à une base pharmaceutiquement acceptable.

14. Composé de formule (I) selon la revendication 1 qui est le N2-({4-[(2-benzoylhydrazino)carbonyl]cyclohexyl}méthyl)-2-naphtalènesulfonamide, ainsi que ses énantiomères, diastéréoisomères, ainsi que ses sels d'addition à un acide ou à une base pharmaceutiquement acceptable.

15. Composé de formule (I) selon la revendication 1 qui est le N1-({4-[(2-benzoylhydrazino)carbonyl]cyclohexyl}méthyl)-1-(2-nitrobenzène)sulfonamide, ainsi que ses énantiomères, diastéréoisomères, ainsi que ses sels d'addition à un acide ou à une base pharmaceutiquement acceptable.

16. Composé de formule (I) selon la revendication 1 qui est le N1-[1-benzyl-2-oxo-2-(2-phénylhydrazino)éthyl]-1-(4-chlorobenzène) sulfonamide, ainsi que ses sels d'addition à un acide ou à une base pharmaceutiquement acceptable.

17. Procédé de préparation des composés de formule (I) caractérisé en ce que l'on utilise comme produit de départ un composé de formule (II) :
H₂N - A - COOH (II)
dans laquelle A est tel que défini dans la formule (I),
qui est condensé en milieu basique sur un dérivé halogéné de formule (III) :
R - Cl (III)
dans laquelle R est tel que défini dans la formule (I),
pour conduire à un composé de formule (IV) :
R - NH - A - COOH (IV)
dans laquelle R et A sont tels que définis précédemment,
composé (IV) qui est condensé, en présence d'un agent de couplage, sur une hydrazine monosubstituée de formule (V),
H₂N - NH - (W)ₙ - Z (V)
dans laquelle n, W et Z sont tels que définis dans la formule (I),
pour conduire aux composés de formule (I) :
R - NH - A - CO - NH - NH - (W)ₙ - Z (I)
dans laquelle R, A, n, W et Z sont tels que définis précédemment,
composé de formule (I) :
- qui peut être, le cas échéant, purifié selon une technique classique de purification
- dont on sépare, le cas échéant, les isomères selon une technique classique de séparation,
- que l'on transforme, si on le souhaite, en ses sels d'addition à un acide ou à une base pharmaceutiquement acceptable.

18. Compositions pharmaceutiques contenant comme principe actif au moins un composé selon l'une quelconque des revendications 1 à 16, seul ou en combinaison avec un ou plusieurs excipients ou véhicules inertes, non toxiques, pharmaceutiquement acceptables.

19. Compositions pharmaceutiques selon la revendication 18 contenant au moins un principe actif selon l'une quelconque des revendications 1 à 16 utiles, comme ligand des récepteurs du Neuropeptide Y, dans le traitement des pathologies liées à des troubles du comportement alimentaire ou de la balance énergétique, telles que le diabète, l'obésité, la boulimie, l'anorexie nerveuse, ou bien dans le traitement de l'hypertension artérielle, de l'anxiété, des dépressions de l'épilepsie, des dysfonctionnements sexuels et des troubles du sommeil.
